# EUROPEAN PATENT SPECIFICATION

(11) **EP 2 945 614 B1**
(45) Date of publication and mention of the grant of the patent: **24.11.2021**
(21) Application number: 14702387.3
(22) Date of filing: 14.01.2014
(51) Int. Cl.: A61K 9/00, A61K 9/28, A61K 31/785

(54) **GASTRO-RETENTIVE SUSTAINED-RELEASE ORAL DOSAGE FORM OF A BILE ACID SEQUESTRANT**
MAGENSAFTRESISTENTEN ORALE RETARD-DOSIERFORM EINES GALLENSÄUREKOMPLEXBILDNERS
FORME POSOLOGIQUE ORALE À LIBÉRATION PROLONGÉE À RÉTENTION GASTRIQUE D'UN AGENT SÉQUESTRANT D'ACIDE BILIAIRE

(30) Priority: 15.01.2013 US 201361752726 P; 11.12.2013 US 201361914804 P
(43) Date of publication of application: 25.11.2015
(60) Divisional application: 21202140.6
(73) Proprietor: Ironwood Pharmaceuticals, Inc., Cambridge, Massachusetts A 02142 (US)
(72) Inventor: SETHURAMAN, Vasu, Waltham, Massachusetts 02451 (US); HEDDEN, David Bruce, Bristol TN 37620-6820 (US); LESKOW, Kristen Marie, Watertown, MA 02472 (US)
(74) Representative: J A Kemp LLP
(86) International application number: PCT/US2014/011450
(87) International publication number: WO 2014/113377

(56) References cited:
- WO-A2-2009/158625
- US-A1- 2007 196 396
- Anonymous: "Drug Delivery, Research Advances", 2007, Nova Science Publishers, XP002723448, pages 190-191
- None

## Description

### CROSS-REFERENCE TO RELATED APPLICATIONS

This application claims priority of United States provisional application 61/752,726, filed January 15, 2013, and United States provisional application 61/914,804, filed December 11,2013.

### TECHNICAL FIELD

The present disclosure relates to gastro-retentive, sustained-release oral dosage forms as described herein comprising a bile acid sequestrant selected from colesevelam and colesevelam hydrochloride, for use in treating disorders of the upper gastrointestinal tract and the throat by administering said dosage forms.

### BACKGROUND

Anatomically, the upper gastrointestinal tract consists of the mouth, a portion of the throat, the esophagus, the stomach and the duodenum, the uppermost part of the small intestine.

The esophagus carries food, liquids, and saliva from the mouth to the stomach by coordinated contractions of its muscular lining. This process is automatic and people are usually not aware of it. Many people have felt their esophagus when they swallow something too large, try to eat too quickly, or drink very hot or very cold liquids. They then feel the movement of the food or drink down the esophagus into the stomach, which may be an uncomfortable sensation.

The muscular layers of the esophagus are normally pinched together at both the upper and lower ends by muscles called sphincters. When a person swallows, the sphincters relax automatically to allow food or drink to pass from the mouth into the stomach. The muscles then close rapidly to prevent the swallowed food or drink from leaking out of the stomach back into the esophagus or into the mouth. These sphincters make it possible to swallow while lying down or even upside-down. When people belch to release swallowed air or gas from carbonated beverages, the sphincters relax and small amounts of food or drink may come back up briefly; this condition is called reflux. The esophagus quickly squeezes the material back into the stomach. This amount of reflux and the reaction to it by the esophagus are considered normal.

While many people are familiar with acid reflux, the backflow of caustic stomach acids into the esophagus, bile reflux is less well known. Bile reflux occurs when bile, a digestive fluid produced in the liver, flows upward (refluxes) from the small intestine into the stomach and then into the esophagus. Bile reflux often accompanies acid reflux, and together they may cause inflammation of the esophageal lining and potentially increased risk of esophageal cancer. *See* AJG (1999) 94(12):3649-3650. Bile reflux may also affect the stomach, where it may cause further inflammation to the stomach (gastritis, which, if untreated, can lead to peptic ulcers)

Unlike acid reflux, bile reflux usually cannot be controlled by changes in diet or lifestyle. Instead, bile reflux is most often managed with certain medications or, in severe cases, with surgery. Neither solution is uniformly effective, however, and some people continue to experience bile reflux even after treatment.

Bile acids are steroid acids found predominantly in the bile of mammals. They are produced in the liver by the oxidation of cholesterol, and are stored in gallbladder and secreted into the intestine in the form of salts. They act as surfactants, emulsifying lipids and assisting with the absorption and digestion of dietary fat and cholesterol.

The principal bile acids are: cholic acid, chenodeoxycholic acid, deoxycholic acid, taurocholic acid, and glycocholic acid. The chemical distinctions between different bile acids are small, depending only on the presence or absence of hydroxyl groups on positions 3, 7, and 12. In humans, the most prevalent bile acids are cholic acid and chenodeoxycholic acid, and their conjugates with taurine and glycine (glycocholate and taurocholate). Some mammals synthesize predominantly deoxycholic acid.

Synthesis of bile acids is a major consumer of cholesterol. The body synthesizes about 800 mg of cholesterol per day and about half of that is used for bile acid synthesis. In total about 20-30 grams of bile acids are secreted into the intestine daily; about 90% of excreted bile acids are reabsorbed (by active transport in the ileum) and recycled. This is referred to as the enterohepatic circulation. Since bile acids are made from endogenous cholesterol, the enterohepatic circulation of bile acids may be disrupted as a way to lower cholesterol. This is the usual therapeutic rationale for administering bile acid sequestrants.

Bile reflux can be difficult to distinguish from acid reflux because the signs and symptoms are similar, and the two conditions frequently occur at the same time. Unlike acid reflux, bile reflux inflames the stomach, often causing a gnawing or burning pain in the upper abdomen. Other signs and symptoms may include: frequent heartburn, *i.e.,* a burning sensation in the chest that sometimes spreads to the throat along with a sour taste in the mouth; nausea; vomiting bile; a cough; or hoarseness.

Bile and stomach acid reflux into the esophagus when the lower esophageal sphincter (LES), malfunctions. The LES separates the esophagus and stomach. Normally, it opens only to allow food to pass into the stomach and then closes tightly. But if the valve relaxes abnormally or weakens, stomach acid and bile can wash back into the esophagus, causing heartburn and ongoing inflammation that may lead to serious complications.

A sticky mucous coating protects the stomach from the corrosive effects of stomach acid, but the esophagus lacks this protection, which is why bile reflux and acid reflux can seriously damage esophageal tissue. And although bile reflux can injure the esophagus on its own — even when the pH of the reflux is neutral or alkaline — the combination of bile and acid reflux seems to be particularly harmful, increasing the risk of complications.

Anatomically, the throat consists of the trachea, the pharynx and the larynx. The throat is separated from the esophagus by the epiglottis, a flap which separates the esophagus from the trachea (the windpipe) and prevents inhalation of food or drink into the lungs. The pharynx, situated immediately inferior to (below) the mouth and the nasal cavity, and superior to the esophagus and larynx is considered part of the digestive system and also the respiratory system. The larynx, commonly called the voice box, is involved in breathing, sound production, and protecting the trachea against food aspiration. It manipulates pitch and volume. The larynx houses the vocal folds (commonly termed the "vocal cords"), which are essential for phonation. The vocal folds are situated just below where the tract of the pharynx splits into the trachea and the esophagus. Due to the anatomical closeness of the upper GI tract and the throat and the fact that both systems are only separated by the epiglottis, bile acid may sometimes reflux into the throat producing additional symptoms.

Disorders and/or symptoms that are believed to be associated with bile reflux, either alone or in combination with acid reflux, include, for instance, heartburn, indigestion, dyspepsia, erosive esophagitis, peptic ulcer, gastric ulcer, esophageal ulcers, esophagitis, laryngitis, pharyngitis, coarse or hoarse voice, and GERD-related pulmonary dysfunction such as coughing and/or asthma. Further complications that are believed to occur as a result of chronic bile reflux are, for instance, gastroesophageal reflux disease, or GERD; Barrett's esophagus; esophageal cancer (e.g., adenocarcinoma) and gastritis.

GERD is a generic term encompassing diseases with various digestive symptoms such as pyrosis, acid regurgitation, obstructed admiration, aphagia, pectoralgia, permeating feeling and the like sensibility caused by reflux in the esophagus and stagnation of gastric contents, duodenal juice, pancreatic juice and the like. The term covers both reflux esophagitis in which erosion and ulcers are endoscopically observed, and esophageal regurgitation-type non-ulcer dyspepsia (NUD) in which no abnormality is endoscopically observed. GERD occurs when the LES does not close properly and stomach contents leak back, or reflux, into the esophagus.

A hiatal hernia may contribute to causing GERD and can happen in people of any age. Other factors that may contribute to GERD include, but are not limited to, alcohol use, being overweight, pregnancy, smoking, Zollinger-Ellison syndrome, hypercalcemia, and scleroderma. Also, certain foods can be associated with reflux events, including, citrus fruits, chocolate, drinks with caffeine, fatty and fried foods, garlic and onions, mint flavorings, spicy foods, and tomato-based foods, like spaghetti sauce, chili, and pizza.

The inner mucosa of the esophagus is lined with non-keratinized stratified squamous epithelium arranged in longitudinal folds. Damage to the lining of the esophagus causes the normal squamous cells that line the esophagus to turn into a type of cell not usually found in humans, called specialized columnar cells. That conversion of cells in the esophagus by the acid reflux is known as Barrett's Esophagus. Although people who do not have heartburn can have Barrett's esophagus, it is found about three to five times more often in people with this condition. Barrett's esophagus does not cause symptoms itself and is important only because it seems to precede the development of a particular kind of cancer— esophageal adenocarcinoma. The risk of developing adenocarcinoma is 30 to 125 times higher in people who have Barrett's esophagus than in people who do not. This type of cancer is increasing rapidly in white men. This increase may be related to the rise in obesity and GERD.

Barrett's esophagus has no cure, short of surgical removal of the esophagus, which is a serious operation. Surgery is recommended only for people who have a high risk of developing cancer or who already have it. Most physicians recommend treating GERD with acid-blocking drugs, since this is sometimes associated with improvement in the extent of the Barrett's tissue. However, this approach has not been proven to reduce the risk of cancer. Treating reflux with a surgical procedure for GERD also does not seem to cure Barrett's esophagus. Several different experimental approaches are under study. One attempts to see whether destroying the Barrett's tissue by heat or other means through an endoscope can eliminate the condition. This approach, however, has potential risks and unknown effectiveness.

Esophageal cancer can occur almost anywhere along the length of the esophagus, but it frequently starts in the glandular cells closest to the stomach (adenocarcinoma). Because esophageal cancer may not be diagnosed until it's quite advanced, the outlook for people with the disease is often poor. The risk of cancer of the esophagus is increased by long-term irritation of the esophagus, such as with smoking, heavy alcohol intake, and Barrett's esophagitis. Thus, there is a link between esophageal cancer and bile reflux and acid reflux. In animal models, bile reflux alone has been shown to cause cancer of the esophagus.

There are numerous medications available that treat heartburn and indigestion. Presently, the main therapies employed in the treatment of GERD and upper GI tract disorders include agents for reducing the stomach acidity, for example by using the histamine H2-receptor antagonists or proton pump inhibitors (PPIs). H2 blockers are drugs that inhibit the production of acid in the stomach. Exemplary histamine H2-receptor antagonists include, for example, cimetidine (as sold under the brand-name TAGAMET HB^{®}), famotidine (as sold under the brand-name PEPCID AC^{®}), nizatidine (as sold under the brand-name AXID AR^{®}), and ranitidine (as sold under the brand-name ZANTAC 75^{®}). Both types of medication are effective in treating heartburn caused by acid reflux and usually eliminate symptoms within a short period of time.

PPIs act by inhibiting the parietal cell H⁺/K⁺ ATPase proton pumps responsible for acid secretion from these cells. PPIs, such as omeprazole and its pharmaceutically acceptable salts are disclosed, for example, in EP 05129, EP 124495 and U.S. Pat. No. 4,255,431.

Despite their well-documented efficacy, PPIs have notable limitations. For example, patients who are non-responsive to treatment with PPI inhibitor alone may be non-responsive because even though the PPI is decreasing acid reflux from the stomach, bile acid from the duodenum is still present. Thus, an improvement of PPI-mediated activity is a well-recognized challenge in gastroenterology and there is a need in the art to address and overcome upper GI tract disorders, as well as related throat disorders as discussed above, that are non-responsive to treatment by administration of PPIs alone.

Accordingly, the development of an effective treatment for pathologies in which bile reflux is involved, either in conjunction with acid reflux or not, would be useful.

WO 2009/158625 relates to the use of at least one bile acid sequestrant for treating gastrointestinal (GI) tract disorders and gastroesophageal reflux disease (GERD)-related respiratory disorders.

### SUMMARY

The present invention addresses this problem by providing an oral dosage form that is able to provide prolonged and steady levels of a bile acid sequestrant selected from colesevelam and colesevelam hydrochloride to the stomach at concentrations which allow for optimal binding of bile acids refluxed from the small intestine into the stomach, thus avoiding bile acid damage to the stomach lining, as well as preventing reflux of stomach bile acids into the esophagus and other parts of the upper GI and the throat, preventing further damage.

Disclosed is a gastro-retentive oral dosage form for sustained release of a bile acid sequestrant selected from colesevelam and colesevelam hydrochloride to the stomach for use in the treatment of a disease of the upper gastrointestinal tract or the throat. The dosage form comprises a bile acid sequestrant selected from colesevelam and colesevelam hydrochloride dispersed in a polymeric matrix. The polymeric matrix comprises poly(alkylene)oxide hydrophilic polymer such that, upon imbibition of gastric fluid, the dosage form swells to a size sufficient to promote gastric retention for a period of time of 3 hours or longer. The bile acid sequestrant is released from the dosage form through erosion of the polymeric matrix over an extended period of time of at least 3 hours.

Also disclosed is a method for making the gastro-retentive oral dosage form disclosed herein. This method comprises combining and blending intragranular components to form an intragranular blend. Next, the intragranular blend is compressed into slugs. These slugs are then milled to form milled granulation. Extragranular components are combined and blended to form an extragranular blend. The extragranular components and milled granulation are then combined and blended to form a dry blend. The extragranular components may be combined and blended at any time prior to their combination with the milled granulation.

In one embodiment, disclosed is the gastric-retentive oral dosage form for use as disclosed herein, comprising administering a therapeutically effective amount of a daily dose of about 100 mg to about 4000 mg of the bile acid sequestrant to a subject in need thereof when the subject is in the fed state.

In one embodiment, disclosed is the gastric-retentive oral dosage form for use as disclosed herein, for use in treating a subject suffering from a disease selected from heartburn, indigestion, dyspepsia, erosive esophagitis, peptic ulcer, gastric ulcer, esophageal ulcers, esophagitis, laryngitis, pharyngitis, coarse voice, gastroesophageal reflux disease (GERD), Barrett's esophagus, gastric cancer, esophageal cancer, gastritis and GERD-related pulmonary dysfunction, comprising administering to the subject a total daily dose of about 100 mg to about 4000 mg of the bile acid sequestrant in the form of the gastric retentive oral dosage form disclosed herein. In some embodiments, the esophageal cancer is adenocarcinoma.

### BRIEF DESCRIPTION OF THE DRAWINGS

Figure 1 demonstrates the drug release rates of various embodiments of the invention.
Figure 2 demonstrates the effect of coating level on drug release rate of an embodiment of the invention.
Figure 3 demonstrates the effect of coating level on drug release rate of an embodiment of the invention.
Figure 4 demonstrates the effect of coating level on drug release rate of an embodiment of the invention.
Figure 5 demonstrates the effect of coating level on drug release rate of an embodiment of the invention.
Figure 6 demonstrates the effect of coating level on drug release rate of an embodiment of the invention.
Figure 7 shows in vitro drug release and disintegration times for embodiments of the invention subsequently used in dogs.
Figure 8 demonstrates in vitro erosion times of various formulations of the invention in dogs.
Figure 9 demonstrates the swellability of tablets of various embodiments of the invention.

### DETAILED DESCRIPTION

Reference will now be made in detail to certain embodiments of the invention, examples of which are illustrated in the accompanying structures, formulae and figures. While the invention will be described in conjunction with the enumerated embodiments, it will be understood that they are not intended to limit the invention to those embodiments.

As employed above and throughout the disclosure, the following terms are provided to assist the reader. Unless otherwise defined, all terms of art, notations and other scientific or medical terms or terminology used herein are intended to have the meanings commonly understood by those of skill in the chemical, pharmaceutical and medical arts. In some cases, terms with commonly understood meanings are defined herein for clarity and/or for ready reference, and the inclusion of such definitions herein should not necessarily be construed to represent a substantial difference over the definition of the term as generally understood in the art unless otherwise indicated.

The terms "drug", "agent", "active pharmaceutical ingredient (API)", "active", "active ingredient (AI)" or "bulk active" are used indistinguishably throughout this disclosure to refer to the substance in a "pharmaceutical product" (i.e., a "medicine" or "medication" or "drug product") that is biologically active. Some medications may contain more than one active ingredient.

As used herein, a "formulation" or "pharmaceutical composition" comprises the API and one or more pharmaceutically acceptable excipients.

The term "dosage form" or "unit dosage form", as used herein, refers to a structure, such as a capsule, a pill, a tablet, an emulsion or syrup, prepared according to a specific procedure from a formulation or pharmaceutical composition that delivers a "dose", or measured quantity of the API to the patient. Dosage forms provide an easily controllable dosage of the drug and enable patient compliance with the prescribed regimen. Various dosage forms may exist for the same compound, since different medical conditions may warrant different routes of administration.

In some embodiments, the dosage form disclosed herein is an oral solid dosage form. In other embodiments, said oral solid dosage form is a tablet. In some embodiments, the dosage form may be administered several times in a period of 24 hours in order to achieve a desired pharmacological effect.

The terms "gastric fluid" and "gastric juice" are used interchangeably throughout the disclosure and refer to the endogenous fluid medium of the stomach, including water and secretions. "Simulated gastric fluid" means any fluid that is generally recognized as providing a useful substitute for authentic gastric fluid in *in-vitro* experiments designed to assess the chemical or biological behavior of substances in the stomach. One such simulated gastric fluid is aqueous 0.1 N HC1, pH 1.2. It will be understood that the term "gastric fluid" or "gastric juice" used throughout the disclosure and claims encompasses both the authentic (i.e. endogenous) gastric fluid and simulated gastric fluids.

The term "gastro-retentive dosage form" denotes dosage forms which effect sustained release of the active ingredient in comparison with conventional dosage forms, such as customary tablets or capsules, while avoiding an undesirably high initial dose, the release being effected continuously over a relatively long period and sustained at a therapeutically effective level by prolonged retention of the dosage form in the stomach.

"Controlled drug delivery systems" supply the drug to the body in a manner precisely controlled to suit the drug and the conditions being treated. The primary aim is to achieve a therapeutic drug concentration at the site of action for the desired duration of time. The term "controlled release" is often used to refer to a variety of methods that modify release of drug from a dosage form. This term includes preparations labeled as "extended release", "delayed release", "modified release" or "sustained release". In general, one can provide for controlled release of the agents described herein through the use of a wide variety of polymeric carriers and controlled release systems including erodible and non-erodible matrices.

"Sustained-release" or "extended-release" preparations are the most common applications of controlled release and throughout this disclosure, both terms will be used interchangeable.

A drug "release rate" as used herein, refers to the quantity of the drug released from a dosage form or pharmaceutical composition per unit time (mg/hr). Drug release rates for drug dosage forms are typically measured as an *in vitro* rate of dissolution, i.e., a quantity of drug released from the dosage form or pharmaceutical composition per unit time measured under appropriate conditions in a suitable fluid. Tests can be performed, for example, at about pH 1.2 (modified simulated gastric fluid, or mSGF) or at about pH 4.5 (the average pH of the stomach after a meal, simulating the fed state). Such testing may also be performed, for instance at 37 °C or 25 °C. Suitable aliquots of the release rate solution (or suspension) are tested to determine the amount of drug released from the dosage form or pharmaceutical composition. A number of analytical techniques, e.g., HPLC, can be used to quantitate the amount of drug released.

As used herein, a "therapeutically or pharmaceutically effective amount" of a drug or agent is an amount of a drug or agent (e.g., a bile acid sequestrant) that, when administered to a subject with a disease or condition will have the intended therapeutic effect, *e.g.,* alleviation, amelioration, palliation or elimination of one or more manifestations of the disease or condition in the subject. The full therapeutic effect does not necessarily occur by administration of one dose and may occur only after administration of a series of doses. Thus, a therapeutically effective amount may be administered in one or more administrations. The term "therapeutically effective amount" as used herein also means that amount of active compound or pharmaceutical agent that elicits the biological or medicinal response in a tissue, system, animal or human that is being sought by a researcher, veterinarian, medical doctor or other clinician. The therapeutically or pharmaceutically effective amount of the compound to be administered will be governed by such considerations, and is the minimum amount necessary to ameliorate, cure or treat the disease or disorder or one or more of its symptoms.

As used herein, a "prophylactically effective amount" of a drug or agent is an amount of a drug or agent (e.g., a bile acid sequestrant) that, when administered to a subject, will have the intended prophylactic effect, *e.g*., preventing or delaying the onset (or reoccurrence) of disease or symptoms, or reducing the likelihood of the onset (or reoccurrence) of disease or symptoms. The full prophylactic effect does not necessarily occur by administration of one dose and may occur only after administration of a series of doses. Thus, a prophylactically effective amount may be administered in one or more administrations. The term "prophylactically effective amount" also refers to an amount effective in preventing or substantially lessening the chances of acquiring a disease or disorder or in reducing the severity of the disease or disorder before it is acquired or reducing the severity of one or more of its symptoms before the symptoms develop. Roughly, prophylactic measures are divided between *primary* prophylaxis (to prevent the development of a disease or symptom) and *secondary* prophylaxis (whereby the disease or symptom has already developed and the patient is protected against worsening of this process).

As used herein, and as would be understood by the person of skill in the art, the recitation of "a compound", is intended to include any solid forms of such compound, including the amorphous form, polymorphs and salts of that compound, or a mixture of any such forms of that compound in any ratio, as it may be applicable.

The term "pharmaceutically acceptable salt" refers to salts prepared from pharmaceutically acceptable non-toxic acids or bases including inorganic acids and bases and organic acids and bases.

When the compounds are basic, salts may be prepared from pharmaceutically acceptable non-toxic acids including inorganic and organic acids. Suitable pharmaceutically acceptable acid addition salts for the compounds of the present disclosure include acetic, benzenesulfonic (besylate), benzoic, camphorsulfonic, citric, ethenesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric acid, p-toluenesulfonic, and the like. When the compounds contain an acidic side chain, suitable pharmaceutically acceptable base addition salts for the compounds of the present disclosure include metallic salts made from aluminum, calcium, lithium, magnesium, potassium, sodium and zinc or organic salts made from lysine, N,N'-dibenzylethylenediamine, chloroprocaine, choline, diethanolamine, ethylenediamine, meglumine (N-methylglucamine) and procaine. For use in medicine, the salts will be pharmaceutically acceptable salts. Other salts may, however, be useful in the preparation of the compounds of the disclosure or of their pharmaceutically acceptable salts. When the compound is basic or contains a sufficiently basic bioisostere, salts may be prepared from pharmaceutically acceptable non-toxic acids, including inorganic and organic acids. Such acids include acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic acid and the like. Particular embodiments include citric, hydrobromic, hydrochloric, maleic, phosphoric, sulfuric and tartaric acids. Other exemplary salts include, but are not limited to, sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucuronate, saccharate, formate, benzoate, glutamate, methanesulfonate, ethanesulfonate, benzenesulfonate, p-toluenesulfonate, and palmoate (i.e., 1,1'-methylene-bis-(2-hydroxy-3-naphthoate)) salts.

In some embodiments, the salts can be prepared *in situ* during the final isolation and purification of the compounds. In other embodiments the salts can be prepared from the free form of the compound in a separate synthetic step.

When the compound is acidic or contains a sufficiently acidic bioisostere, suitable "pharmaceutically acceptable salts" refers to salts prepared form pharmaceutically acceptable non-toxic bases including inorganic bases and organic bases. Salts derived from inorganic bases include aluminum, ammonium, calcium, copper, ferric, ferrous, lithium, magnesium, manganic salts, manganous, potassium, sodium, zinc and the like. Particular embodiments include ammonium, calcium, magnesium, potassium and sodium salts. Salts derived from pharmaceutically acceptable organic non-toxic bases include salts of primary, secondary and tertiary amines, substituted amines including naturally occurring substituted amines, cyclic amines and basic ion exchange resins, such as arginine, betaine, caffeine, choline, N, N¹-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine tripropylamine, tromethamine and the like.

The preparation of the pharmaceutically acceptable salts described herein and other typical pharmaceutically acceptable salts is more fully described by Berg et al., "Pharmaceutical Salts," J. Pharm. Sci., 1977:66:1-19.

Bile acid sequestrants are drugs that bind bile acids in the small intestine and carry them out of the body. This causes the body to use more cholesterol to make more bile acids, which are secreted into the small intestine, bound to bile acid sequestrants, and carried out of the body. The end result is lower cholesterol levels. Bile acid sequestrants also prevent absorption of some dietary cholesterol.

Bile acid sequestrants currently approved for human use are polymeric compounds which serve as ion exchange resins. Bile acid sequestrants exchange anions such as chloride ions for bile acids. By doing so, they bind bile acids and sequester them from enterohepatic circulation. Since bile acid sequestrants are large polymeric structures, they are not absorbed from the gut into the bloodstream. Thus, bile acid sequestrants, along with any bile acids bound to the drug, are excreted via the feces after passage through the gastrointestinal tract. Exemplary bile acid sequestrants include, for example, cholestyramine (as sold under the brand-name QUESTRAN^{®}), colesevelam (as sold under the brand-name WELCHOL^{®}), Selevamer (Rinogel®) and colestipol (as sold under the brand-name COLESTID^{®}), and pharmaceutically acceptable salts thereof.

Gastric-retained oral dosage forms make use of one or more hydrophilic polymers which swell upon intake of water from gastric fluid. When administered in the fed mode, when the diameter of the pyloric sphincter is contracted and reduced, these dosage forms will swell to a size that is too large to pass through the pyloric sphincter and are retained in the stomach for a minimum of 3 hours or more. Although gastro-retentive sustained-release dosage forms for oral delivery of sparingly soluble drugs and insoluble matter have been described, gastric-retentive sustained-release dosage forms for oral administration of a polymeric active pharmaceutical ingredient (API) such as a bile-acid sequestrant have not been described.

It is an object of the present invention to provide an oral dosage form which is able to provide prolonged and steady levels of a bile acid sequestrant selected from colesevelam and colesevelam hydrochloride to the stomach at concentrations which allow for optimal binding of bile acids refluxed from the small intestine into the stomach, thus avoiding bile acid damage to the stomach lining, as well as preventing reflux of stomach bile acids into the esophagus and other parts of the upper GI and the throat, preventing further damage.

The transit time through the intestinal tract often limits the amount of a drug that is available for delivery to its most efficient site of action. To counter this issue, oral administration of sparingly soluble drugs usually involves frequent dosing, often 3 or more times per day. In addition, drugs that are insoluble cannot readily be delivered by either solution-diffusion or membrane-controlled drug delivery systems. In contrast, the inventors have found that erodible, gastric retentive dosage forms of polymeric bile acid sequestrants allow for the sustained delivery of bile acid sequestrants to the stomach, wherein they can most efficiently bind excess bile acids that otherwise would be free to reflux into the esophagus and other areas of the upper GI and the throat.

In a first aspect, the invention is a gastro-retentive oral dosage form for sustained release of a bile acid sequestrant to the stomach for use in the treatment of a disease of the upper gastrointestinal tract or the throat, wherein said dosage form comprises a bile acid sequestrant selected from colesevelam and colesevelam hydrochloride dispersed in a polymeric matrix comprising poly(alkylene)oxide, wherein the polymeric matrix comprises a poly(alkylene)oxide hydrophilic polymer such that, upon imbibition of gastric fluid, said dosage form swells to a size sufficient to promote gastric retention for a period of time of 3 hours or longer and wherein the bile acid sequestrant is released from the dosage form through erosion of the polymeric matrix over an extended period of time.

The sustained-release oral dosage forms here provided gradually erode while retained in the stomach over a period of several hours with the erosion commencing upon contact with the gastric fluid and the active ingredient being released to the stomach at a rate that depends on the erosion rate of the polymeric matrix.

The bile acid sequestrant is selected from colesevelam or colesevelam hydrochloride.

The bile acid sequestrants described herein can be incorporated into an erodible polymeric matrix controlled release device. By an "erodible matrix" is meant an aqueous-erodible or water-swellable or aqueous-soluble in the sense of being either erodible or swellable or dissolvable (or combinations of these properties) in pure water or requiring the presence of an acid or base to ionize the polymeric matrix sufficiently to cause erosion or dissolution (e.g. gastric fluid).

The amount of active ingredient that may be combined with the carrier material to produce a single dosage form will vary depending upon the subject treated and the particular mode of administration. For example, a time-release formulation intended for oral administration to humans may contain approximately 100 to 1000 mg of active material compounded with an appropriate and convenient amount of carrier material which may vary from about 5 to about 95% of the total compositions (weight: weight).

In some embodiments, the gastro retentive oral dosage forms of the invention comprise a dose of bile acid sequestrant between 100 and 750 mg. In other embodiments, the dose of bile acid sequestrant in a gastro-retentive oral dosage form of the invention is between about 400 mg and about 600 mg. In still other embodiments, the dose of bile acid sequestrant is said gastro-retentive oral dosage form is about 500 mg.

In some embodiments, the gastro retentive oral dosage forms of the invention, comprise an amount of the bile acid sequestrant which is up to 75 % of the total composition weight (weight: weight). In other embodiments, the amount of the bile acid sequestrant is up to 50 % of the total composition weight (weight: weight).

When contacted with the aqueous environment of use (e.g., gastric fluid), the erodible polymeric matrix imbibes water and forms an aqueous-swollen gel or matrix that entraps the bile acid sequestrant. The aqueous-swollen matrix gradually erodes, swells, disintegrates and/or dissolves in the environment of use, thereby controlling the release of the bile acid sequestrant to the environment of use.

An essential ingredient of this water-swollen matrix is poly(alkylene)oxide. Hydrophilic, water-swellable, erodible, or soluble polymer may generally be described as an "osmopolymer", "hydrogel" or "water-swellable" polymer. More than one of such polymers may be combined in a dosage form of the invention in order to achieve gastric-retention as well as the desired erosion rate.

The terms "hydrophilic" and "hydrophobic" are generally defined in terms of a partition coefficient P, which is defined as the ratio of the equilibrium concentration of a compound in an organic phase to that in an aqueous phase. A hydrophilic compound has a P value of less than 1.0, typically less than about 0.5, wherein P is the partition coefficient of the compound between octanol and water. A hydrophobic compound will generally have a P value greater than about 1.0, typically greater than about 5.0. The polymeric carriers herein are hydrophilic, and thus are compatible with aqueous fluids such as those present in the human body, in particular in the stomach.

The term "polymer", as used herein, refers to a molecule containing a plurality of covalently attached monomer units, and includes branched, dendrimic and star polymers as well as linear polymers. The term includes both homopolymers and copolymers, for example random copolymers, block copolymers, and graft copolymers, as well as uncrosslinked polymers and slightly to moderately to substantially cross-linked polymers, as well as two or more inter-penetration cross-linked networks.

The term "swellable polymer", as used herein, refers to a polymer that will swell in the presence of a fluid. It is understood that a given polymer may or may not swell when present in a defined drug formulation. Accordingly, the term "swellable polymer" defines a structural feature of a polymer which is dependent upon the composition in which the polymer is formulated. Whether or not the polymer swells in the presence of fluid will depend on a number of factors, including the specific type of polymer and the percentage of that polymer in a particular formulation.

The terms "swellable" or "bioerodible" (or simply "erodible") are used to refer to the polymers used in the present dosage forms, with "swellable polymers" being those that are capable of absorbing water and physically swelling as a result, with the extent to which a polymer can swell being determined by the molecular weight or degree of cross-linking (for cross-linked polymers), and "bioerodible" or "erodible" polymers referring to polymers that slowly dissolve and/or gradually hydrolyze in an aqueous fluid, and/or that physically disentangle or undergo chemical degradation of the chains themselves, as a result of movement within the stomach or GI tract. Some hydrophilic polymers have the property of being both swellable and erodible simultaneously. Other hydrophilic polymers are only erodible.

Polymers suitable for achieving the desired gastro-retentive and sustained-release profiles of the dosage forms of the invention have the property of swelling as a result of imbibing water from the gastric fluid, and gradually eroding over a time period of several hours. Since erosion of the polymer results from the interaction of the fluid with the surface of the dosage form, erosion initiates more or less simultaneously with the swelling process. While erosion and swelling may occur at the same time, the rate for achieving maximum swelling should be faster than the rate the dosage form fully erodes to achieve the desired release profile.

Such polymers may be linear, branched, or cross linked. The polymers may be homopolymers or copolymers.

The term "polyethylene oxide" or "PEO" refers to a polyethylene oxide polymer that has a wide range of molecular weights. PEO is a linear polymer of unsubstituted ethylene oxide and has a wide range of viscosity-average molecular weights. Examples of commercially available PEOs and their approximate molecular weights (in grams/mole or Daltons) are: POLYOX® NF, grade WSR coagulant, approximate molecular weight 5 million; POLYOX® grade WSR 301, approximate molecular weight 4 million; POLYOX® grade WSR 303, approximate molecular weight 7 million; POLYOX® grade WSR N60-K, approximate molecular weight 2 million; POLYOX® grade WSR N-80K, approximate molecular weight 200,000.

The poly(alkylene)oxide of the gastro-retentive oral dosage forms described herein is a swellable and erodible polymer.

In the gastro-retentive, oral dosage form of the present invention, said polymer is a polyalkylene oxide. In some embodiments, at least one of the one or more hydrophilic polymers is a polyethylene oxide (PEO). In still other embodiments, the at least one hydrophilic polymer is a polyethylene oxide having a molecular weight of about 2,000,000 to 4,000,000 Daltons.

In some embodiments, the poly(ethylene)oxide is present in the unit dosage form in an amount ranging from 40 weight percent ratio to 75 weight percent ratio. In some embodiments, the poly(ethylene)oxide is present in the unit dosage form in an amount ranging from 40 weight percent ratio to 60 weight percent ratio. In some embodiments, the poly(ethylene)oxide is present in the unit dosage form in an amount ranging from 45 weight percent ratio to 55 weight percent ratio. In some embodiments, the poly(ethylene)oxide is present in the unit dosage form in an amount ranging from 45 weight percent ratio to 60 weight percent ratio. In some embodiments, the poly(ethylene)oxide is present in the unit dosage form in an amount ranging from 40 weight percent ratio to 50 weight percent ratio. In some embodiments, the poly(ethylene)oxide is present in the unit dosage form in an amount ranging from 50 weight percent ratio to 60 weight percent ratio. In some embodiments, the poly(ethylene)oxide is present in the unit dosage form in an amount ranging from 47 weight percent ratio to 53 weight percent ratio.

The dosage form may further comprise as a hydrophilic polymer a cellulose, synthetic polymers derived from vinyl, acrylate, methacrylate, urethane, ester and oxide monomers, derivatives of naturally occurring polymers such as polysaccharides (e.g. chitin, chitosan, dextran and pullulan; gum agar, gum arabic, gum karaya, locust bean gum, gum tragacanth, carrageenans, gum ghatti, guar gum, xanthan gum and scleroglucan), starches (e.g. dextrin and maltodextrin, corn-starch- unmodified or pregelatinized-), hydrophilic colloids (e.g. pectin), phosphatides (e.g. lecithin), alginates (e.g. ammonium alginate, sodium, potassium or calcium alginate, propylene glycol alginate), gelatin, collagen, and cellulosics. Cellulosics are cellulose polymer that has been modified by reaction of at least a portion of the hydroxyl groups on the saccharide repeat units with a compound to form an ester-linked or an ether-linked substituent. For example, the cellulosic ethyl cellulose has an ether linked ethyl substituent attached to the saccharide repeat unit, while the cellulosic cellulose acetate has an ester linked acetate substituent.

Cellulosics may comprise aqueous-soluble and aqueous-erodible cellulosics can include, for example, methylethyl cellulose (MEC), carboxymethyl cellulose (CMC), CMEC, hydroxyethyl cellulose (HEC), hydroxypropyl cellulose (HPC), cellulose acetate (CA), cellulose propionate (CP), cellulose butyrate (CB), cellulose acetate butyrate (CAB), CAP, CAT, hydroxypropyl methyl cellulose (HPMC), HPMCP, HPMCAS, hydroxypropyl methyl cellulose acetate trimellitate (HPMCAT), and ethylhydroxy ethylcellulose (EHEC). In certain embodiments, the cellulosics comprises various grades of low viscosity (MW less than or equal to 50,000 Daltons, for example, the Dow Methocel™ series E5, E15LV, E50LV and K100LY) and high viscosity (MW greater than 50,000 Daltons, for example, E4MCR, E10MCR, K4M, K15M and K100M and the Methocel™ K series) HPMC. Other commercially available types of HPMC include the Shin Etsu Metolose 90SH series.

The erodible matrix material may also include other materials, such as pullulan, polyvinyl pyrrolidone (povidone), polyvinyl alcohol, polyvinyl acetate, glycerol fatty acid esters, polyacrylamide, polyacrylic acid, copolymers of ethacrylic acid or methacrylic acid (EUDRAGIT®, Rohm America, Inc., Piscataway, New Jersey) and other acrylic acid derivatives such as homopolymers and copolymers of butylmethacrylate, methylmethacrylate, ethylmethacrylate, ethylacrylate, (2-dimethylaminoethyl) methacrylate, and (trimethylaminoethyl) methacrylate chloride.

In certain embodiments, the pharmaceutical dosage form is retained in the stomach for a period of 3-24 hours (e.g., 3, 4, 5, 6, 7, 8, 9, 10, 11, 12, 13, 14, 15, 16, 17, 18, 19, 20, 21, 22, 23, or 24 hours). For purposes of this disclosure, when the term "at least X hours" is used, the term should be understood to mean X hours to 24 hours, inclusive; to be perfectly clear, the term "at least 3 hours" is understood to mean 3 hours to 24 hours, inclusive. In some embodiments, the pharmaceutical dosage form is retained in the stomach for at least 3 hours. In some embodiments, the pharmaceutical dosage form is retained in the stomach for at least 4 hours. In some embodiments, the pharmaceutical dosage form is retained in the stomach for at least 6 hours. In some embodiments, the pharmaceutical dosage form is retained in the stomach for at least 7 hours. In some embodiments, the pharmaceutical dosage form is retained in the stomach for at least 8 hours. In some embodiments, the pharmaceutical dosage form is retained in the stomach for at least 10 hours. In some embodiments, the pharmaceutical dosage form is retained in the stomach for at least 11 hours. In some embodiments, the pharmaceutical dosage form is retained in the stomach for at least 12 hours.

The polymer matrix erodes in the stomach during a period of drug release. For purposes of this disclosure, when the term "at least X hours" is used, the term should be understood to mean X hours to 24 hours, inclusive; to be perfectly clear, the term "at least 3 hours" is understood to mean 3 hours to 24 hours, inclusive. In some embodiments, the period of drug release is at least four hours. In some embodiments, the period of drug release is at least six hours. In some embodiments, the period of drug release is at least eight hours. In some embodiments, the period of drug release is at least ten hours. In some embodiments, the period of drug release is at least eleven hours. In some embodiments, the period of drug release is at least twelve hours. In some embodiments, the polymer matrix erodes in the stomach during a period that starts after drug release has started. Erosion may not occur during the full period of drug release. For example, at earlier time points as the matrix swells some of the drug release will occur just based on swelling with minimal erosion.

The rate of drug release from the gastro-retentive dosage form disclosed herein may be measured *in vitro* in acetate buffer at pH 4.5, using a USP Type II (paddle) apparatus with the tablets placed in sinkers. In some embodiments, between 30-85% of the drug has been released from the dosage form after 10 hours. In some embodiments, between 50-85% of the drug has been released from the dosage form after 10 hours. In some embodiments, between 60-85% of the drug has been released from the dosage form after 10 hours. In some embodiments, between 65-85% of the drug has been released from the dosage form after 10 hours. In some embodiments, between 70-85% of the drug has been released from the dosage form after 10 hours. In some embodiments, between 75-100% of the drug has been released from the dosage form after 12 hours. In some embodiments, between 75-100% of the drug has been released from the dosage form after 16 hours. In some embodiments, between 85-100% of the drug has been released from the dosage form after 12 hours. In some embodiments, between 85-100% of the drug has been released from the dosage form after 16 hours. In some embodiments, between 80-100% of the drug has been released from the dosage form after 20 hours. In some embodiments, between 90-100% of the drug has been released from the dosage form after 20 hours. In some embodiments, about 90 to 100% of the drug has been released from the dosage form after 24 hours.

In certain embodiments, the gastro-retentive oral dosage swells to a size that is at least 110 % of the original size by 30 minutes. In other embodiments, it swells to a size that is at least 130 % of the original size by 2 hours. In other embodiments, it swells to a size that is at least 140 % of the original size by 2 hours.

In some embodiments, the gastro-retentive oral dosage form comprises two polymers, both of them being swelling and hydrophilic erodible polymers. In some embodiments, the first of said polymers is a PEO and the second of said polymers is a cellulose.

In some embodiments, the gastro-retentive oral dosage form comprises two polymers, one of them being a poly(alkylene)oxide swelling hydrophilic erodible polymer and the other one being a swelling and non-hydrophilic non-erodible polymer. In some embodiments, the swelling and hydrophilic erodible polymers selected from a PEO. In some embodiments, the swelling and non-hydrophilic non-erodible polymer is 600 mg PolyOx Coag.

In still other embodiments, the gastro-retentive oral dosage form comprises two polymers, one of them being a swelling hydrophilic erodible polymer and the other one being a non-swelling hydrophilic erodible polymer. In some embodiments, the swelling and hydrophilic erodible polymer is selected from a PEO or a cellulose. In some embodiments, the non-swelling hydrophilic erodible polymer is selected from ethylcelullose, cellulose acetate, hydroxypropyl methylcellulose acetate succinate or Eudragit RSPO.

In certain embodiments, the dosage form may additionally contain suitable diluents, glidants, lubricants, acidulants, stabilizers, fillers, binders, plasticizers or release aids and other pharmaceutically acceptable excipients.

In some embodiments, the gastro-retentive oral dosage form of the invention comprises a filler or compressing agent selected from microcrystalline cellulose, lactose, starch, maltodextrins and dibasic calcium phosphate. In other embodiments, the filler or compression agent is a microcrystalline cellulose.

In some embodiments, the gastro-retentive oral dosage form of the invention comprises a glidant selected from silicon dioxide and talc.

In some embodiments, the gastro-retentive oral dosage form of the invention comprises a lubricant selected from magnesium stearate, stearic acid, sodium stearyl fumarate. In other embodiments, the lubricant is magnesium stearate.

In some embodiments, the gastro-retentive oral dosage form of the invention is comprised of a core and a coating. In some embodiments, the coating represents between 5 % and 7.5 % of the total weight of the formulation (weight: weight).

In some embodiments, the coating is comprised of a binder and a plasticizer. In some embodiments the plasticizer is an acetylated glyceride. In some embodiments, the binder is selected from hydroxypropylmethylcellulose, CAP, HPMCAS, HPMCP or ethylcellulose.

### Dosage Form preparation

In a second aspect, the current disclosure relates to a method for manufacturing a gastro-retentive sustained-release dosage form comprising at least one bile acid sequestrant, such as those that have been described in the previous section.

The active agents used in the dosage forms of the present disclosure can be formulated in accordance with methods that are standard in the art (see e.g., Remington: the Science and Practice of Pharmacy 21st Ed. 2005, University Sciences in Philadelphia Pa.) or Developing Solid Oral Dosage Forms - Pharmaceutical Theory and Practice, 1st Ed; Academic Press; Burlington, MA.

During the process, the drug may be mixed with conventional excipients, carriers, buffers, flavoring agents, etc. Typical carriers include, but are not limited to: water; salt solutions; alcohols; gum arabic; vegetable oils; benzyl alcohols; polyethylene glycols; gelatin; carbohydrates, such as lactose, amylose or starch; magnesium stearate; talc; silicic acid; paraffin; perfume oil; fatty acid esters; hydroxymethylcellulose; polyvinyl pyrrolidone; etc. Pharmaceutical preparations can be sterilized and, if desired, mixed with auxiliary agents such as: lubricants; preservatives; disintegrants; stabilizers; wetting agents; emulsifiers; salts; buffers; natural or artificial coloring agents; natural or artificial flavoring agents; or aromatic substances.

Compressed tablets may be prepared by compressing in a suitable machine the active ingredient in a free-flowing form such as a powder or granules, optionally mixed with a binder, lubricant, inert diluent, preservative, surface active or dispersing agent. Molded tablets may be made by molding in a suitable machine a mixture of the powdered active ingredient moistened with an inert liquid diluent. Standard methods for tablet preparation include direct compression, dry granulation with roller compaction, dry granulation with slugging and wet granulation. These methods are well known to those skilled in the art.

"Granulation", as used herein, is defined as the process in which primary powder particles are made to adhere to form larger, multi-particle entities called granules. It is the process of collecting particles together by creating bonds between them. Bonds are formed by compression ("dry granulation") or by using a binding agent ("wet granulation"). Granulation is extensively used in the manufacturing of tablets. The granulation process generally combines one or more powder particles and forms a granule that will allow tableting process to be within required limits. This way a predictable and repeatable process is possible and quality tablets or pellets can be produced using tableting equipment.

Dry granulation can be conducted under two processes; either a large tablet ("slug") is produced in a heavy duty tableting press ("slugging") or the powder is squeezed between two rollers to produce ribbons of materials ("roller compaction"). These materials (i.e., the slugs or the ribbons) are then milled to provide the "granules".

In accordance with the present disclosure, when the ingredients are incorporated prior to granulation, they are referred to as "intragranular", i.e., within the granule. When the ingredients are incorporated after granulation, they are referred to as "extragranular".

The enteric coating surrounding the core may be applied using standard coating techniques. Materials used to form the enteric coating may be dissolved or dispersed in organic or aqueous solvents and may include one or more of the following: methacrylic acid copolymers; shellac; hydroxypropylmethylcellulose phthalate; polyvinyl acetate phthalate; hydroxypropylmethylcellulose trimellitate; carboxymethylcellulose; cellulose acetate phthalate; or other suitable enteric coating polymers. The pH at which the enteric coat will dissolve can be controlled by the polymer or combination of polymers selected and/or ratio of pendant groups. For example, dissolution characteristics of the coating can be altered by the ratio of free carboxyl groups to ester groups. Enteric coating layers may also contain pharmaceutical plasticizers such as: triethyl citrate; dibutyl phthalate; triacetin; polyethylene glycols; polysorbates; acetylated glycerides, etc. Additives such as dispersants, colorants, anti-adhering, taste-masking and anti-foaming agents may also be included.

In some embodiments, the coating of the unit dosage form of the invention comprises a microcrystalline cellulose and an acetylated glyceride.

In some embodiments, the gastro-retentive sustained release dosage forms of the invention can be prepared by a process as described below. Intragranular components are combined and blended to form an intragranular blend. In some instances, the bile acid sequestrant (active ingredient) is one of the intragranular components. The intragranular components may further include fillers or compression aids, such as microcrystalline cellulose, and/or lubricants, such as magnesium stearate. The intragranular blend is compressed into slugs, and the slugs are milled to form milled granulation. The yield for the milled granulation is calculated so that the desired amounts of the extragranular components to be used can be determined. Extragranular components are combined and blended to form an extragranular blend. In some instances, the hydrophilic polymer is one of the extragranular components. There may be more than one hydrophilic polymer present. In some instances, the hydrophilic polymer may be comprised of polyalkylene oxide, such as polyethylene oxide. The extragranular components may include fillers or compression aids, such as microcrystalline cellulose; binders or drug release aids, such as trehalose or hydroxypropylmethylcellulose; plasticizers, such as diacetylated monoglyceride; and/or lubricants, such as magnesium stearate. The extragranular components and milled granulation are then combined and blended to form a dry blend. The extragranular components may be combined and blended at any time prior to their combination with the milled granulation; that is the extragranular components may be combined and blended before the intragranular components are combined and blended, or vice versa.

In some embodiments, the dry blend may be compressed into one or more tablets. In other embodiments, the tablets may be coated with an outer layer (coating). In some embodiments, the coating may be 3:1 HPMC (Grade - E50 Premium LV): diacetylated monoglycerides, NF Grade-(Myvacet 9-45K).

The dosage forms of the invention may be packaged for use in a variety of ways depending upon the method used for administering the drug. Generally, an article for distribution includes a container having deposited therein the pharmaceutical dosage form. Suitable containers are well-known to those skilled in the art and include materials such as bottles (plastic and glass), sachets, ampoules, plastic bags, metal cylinders, and the like. The container may also include a tamper-proof assemblage to prevent indiscreet access to the contents of the package. In addition, the container has deposited thereon a label that describes the contents of the container. The label may also include appropriate warnings.

### Therapeutic methods

The gastro-retentive, sustained-release oral dosage forms comprising at least one bile acid sequestrant that are herein described are for use in treating disorders of the upper gastrointestinal (GI) tract and the throat. In some instances the disorders of the upper GI are esophageal disorders. In certain embodiments, the patient may be suffering from (or is susceptible to developing) an upper GI tract or throat disorder selected from one or more of: Gastroesophageal reflux disease, or GERD, including non-responsive GERD; Barrett's esophagus; esophageal cancer, gastritis, heartburn, indigestion, dyspepsia, erosive esophagitis, peptic ulcer, gastric ulcer, gastric cancer, esophageal ulcer, esophagitis, laryngitis, pharyngitis, coarse voice, and GERD-related pulmonary dysfunction such as coughing and/or asthma. In some embodiments, the upper GI tract or throat disorder is GERD or dyspepsia. In some embodiments, the upper GI tract or throat disorder is GERD. In some embodiments, the upper GI tract or throat disorder is dyspepsia.

As used herein "non-responsive GERD" refers to chronic reflux disorders that do not respond to current therapies used to treat such conditions. Such therapies include, for example, administration of proton pump inhibitors, H₂ blockers, and various antacids that are well known in the art.

In some embodiments, disclosed is the gastric-retentive oral dosage form for use as disclosed herein, comprising administering a therapeutically effective amount of a daily dose of 100 mg to 4000 mg of the bile acid sequestrant selected from colesevelam and colesevelam hydrochloride to a subject in need thereof. In some embodiments, the daily dose of the bile acid sequestrant is 100 mg to 2500 mg. In some embodiments, the daily dose of the bile acid sequestrant is 500 mg to 4000 mg. In some embodiments, the daily dose of the bile acid sequestrant is 500 mg to 2500 mg. In some embodiments, the daily dose of the bile acid sequestrant is 1000 mg to 4000 mg. In some embodiments, the daily dose of the bile acid sequestrant is 1000 mg to 3000 mg. In some embodiments, the daily dose of the bile acid sequestrant is 2000 mg to 4000 mg. In some embodiments, the daily dose of the bile acid sequestrant is 1500 mg to 3000 mg. In some embodiments, the daily dose of the bile acid sequestrant is 200 mg to 3000 mg. In some embodiments, the daily dose of the bile acid sequestrant is 2000 mg to 2500 mg. In these embodiments, the subject is administered the gastric-retentive oral dosage form disclosed herein when the subject is in the fed state.

In some embodiments, the dose of bile acid sequestrant described herein is administered up to 4 times in a 24-hour period. In other embodiments, it is administered up to 3 times in a 24-hour period. In other embodiments, it is administered 1 or 2 times a day. In still other embodiments, it is administered once a day.

In some embodiments, the dose of bile acid sequestrant described herein is administered with one or more meals. In some embodiments, the dose of bile acid sequestrant described herein is administered at bedtime. In some embodiments, the dose of bile acid sequestrant described herein is administered with one or more meals and at bedtime. In some embodiments, the dose of bile acid sequestrant described herein is administered before or after one or more meals. In some embodiments, the dose of bile acid sequestrant described herein is administered with a meal. In some embodiments, the dose of bile acid sequestrant described herein is administered up to 30 minutes after the meal. In some embodiments, the dose of bile acid sequestrant described herein is administered up to 5 minutes before the meal.

Disclosed is the use of a gastro-retentive oral dosage form described herein for sustained release of a bile acid sequestrant to the stomach for use in the treatment of a disease of the upper gastrointestinal tract or the throat.

In some embodiments, disclosed is a gastro-retentive oral dosage form described herein for use in treating a subject suffering from a disease selected from heartburn, indigestion, dyspepsia, erosive esophagitis, peptic ulcer, gastric ulcer, esophageal ulcers, esophagitis, laryngitis, pharyngitis, coarse voice, gastroesophageal reflux disease (GERD), Barrett's esophagus, gastric cancer, esophageal cancer (e.g., adenocarcinoma), gastritis and GERD-related pulmonary dysfunction. In some embodiments, a total daily dose of about 100 mg to about 4000 mg of a bile acid sequestrant in the form of the gastric retentive oral dosage form is administered to the subject.

The terms, "disease", "disorder" and "condition" may be used interchangeably here to refer to a medical or pathological condition or symptom that is believed to be the result of bile reflux.

As used herein, the terms "subject" and "patient" are used interchangeably. The terms "subject" and "patient" refer to an animal (e.g., a bird such as a chicken, quail or turkey, or a mammal), specifically a "mammal" including a non-primate (e.g., a cow, pig, horse, sheep, rabbit, guinea pig, rat, cat, dog, and mouse) and a primate (e.g., a monkey, chimpanzee and a human), and more specifically a human. In some embodiments, the subject is a non-human animal such as a farm animal (e.g., a horse, cow, pig or sheep), or a pet (e.g., a dog, cat, guinea pig or rabbit). In some embodiments, the subject is a human.

A "susceptible individual" or "a patient in need thereof' is an individual who suffers from, is suffering from, or is likely to or predisposed to suffer from an upper GI tract or a throat disorder that is believed to be result of bile reflux. In humans, and as used herein, these conditions may include, for example heartburn, indigestion, dyspepsia, erosive esophagitis, peptic ulcer, gastric ulcer, esophageal ulcers, esophagitis, laryngitis, pharyngitis, coarse voice, and GERD-related pulmonary dysfunction such as coughing and/or asthma. Further complications that are believed to occur as a result of bile reflux are, for instance, Gastroesophageal reflux disease, or GERD; Barrett's esophagus; esophageal cancer (e.g., adenocarcinoma) and gastritis. In animals these conditions may include, for example, peptic ulcer of the forestomach.

The term "biological sample", as used herein, refers to an *in vitro* or *ex vivo* sample, and includes, without limitation, cell cultures or extracts thereof; biopsied material obtained from a mammal or extracts thereof; blood, saliva, urine, faeces, semen, tears, lymphatic fluid, ocular fluid, vitreous humour, or other body fluids or extracts thereof.

"Treat", "treating" or "treatment" with regard to a disorder or disease refers to alleviating or abrogating the cause and/or the effects of the disorder or disease. Treatment can involve administering a compound described herein to a patient diagnosed with a disease, and may involve administering the compound to a patient who does not have active symptoms. Conversely, treatment may involve administering the compositions to a patient at risk of developing a particular disease, or to a patient reporting one or more of the physiological symptoms of a disease, even though a diagnosis of this disease may not have been made.

As used herein, "treating" or "treatment of' a condition or subject refers to taking steps to obtain beneficial or desired results, including clinical results. For purposes of this disclosure, beneficial or desired clinical results include, but are not limited to, alleviation or amelioration of one or more disease, symptom, or condition that arises as a result of bile refluxing into the upper GI tract or the throat.

The terms "administer", "administering" or "administration" in reference to a dosage form of the invention refers to the act of introducing the dosage form into the system of subject in need of treatment. When a dosage form of the invention is given in combination with one or more other active agents (in their respective dosage forms), "administration" and its variants are each understood to include concurrent and/or sequential introduction of the dosage form and the other active agents.

Administration of any of the described dosage forms includes parallel administration, co-administration or sequential administration, in which the therapies are administered at approximately the same time, *e.g*., within about a few seconds to a few hours of one another.

The term "fed mode", as used herein, refers to a state which is typically induced in a patient by the presence of food in the stomach, the food giving rise to two signals, one that is said to stem from stomach distension and the other a chemical signal based on food contents in the stomach. It has been determined that once the fed state is induced, larger particles are retained in the stomach for a longer period of time than smaller particles. The fed mode is induced by nutritive materials entering the stomach upon the ingestion of food. Initiation of the fed state is accompanied by a rapid and profound change in the motor pattern of the upper GI tract, over a period of 30 seconds to one minute. The change is observed almost simultaneously at all sites along the GI tract and occurs before the stomach contents have reached the distal small intestine. Once the fed state is established, the stomach generates 3-4 continuous and regular contractions per minute, similar to those in the fasted mode but with about a quarter to half the amplitude (Force). The pylorus is partially opened, causing a sieving effect in which liquids and small particles flow continuously from the stomach into the intestine while indigestible particles greater in size than the pyloric opening are retropelled and retained in the stomach. This effect causes the stomach to retain particles exceeding about 1 cm in size for approximately 4 to 8 hours or more.

Administration of a dosage form "with a meal", as used herein, refers to administration during or after the ingestion of food or drink. When the dosage form is administered after a meal, it may be administered about 1, 2, 3, 4, 5, 10, 15 or up to 30 minutes after completion of a meal. In some embodiments, the dosage form may be administered up to 5 minutes before the meal.

In another aspect, the patient has a genetic predisposition to developing a bile reflux related disorder. In another aspect, a dosage form herein described, is administered to a patient in order to prevent or minimize damage to the upper GI tract or the throat.

In one embodiment, the treatment of the invention is a preventative or "pre-emptive" measure to a patient, specifically a human, having a predisposition (e.g. a genetic predisposition) to developing a disease, disorder or symptom believed to be the result of bile reflux.

In other embodiments, the treatment of the invention is a preventative or "pre-emptive" measure to a patient, specifically a human, suffering from a disease, disorder or condition that makes him at risk of developing a bile reflux related disorder or symptom.

The gastric-retentive sustained-release oral dosage forms here disclosed are also useful for use in veterinary treatment of companion animals, exotic animals and farm animals, including, without limitation, dogs, cats, mice, rats, hamsters, gerbils, guinea pigs, rabbits, horses, pigs and cattle.

### Combination Therapies

The gastric-retentive, sustained-release oral dosage forms comprising at least one bile acid sequestrant selected from colesevelam and colesevelam hydrochloride described herein can be used in combination therapy with one or more additional therapeutic agents. For combination treatment with more than one active agent, where the active agents may be in separate dosage forms, the active agents may be administered separately or in conjunction. In addition, the administration of one agent may be prior to, concurrent to, or subsequent to the administration of the other agent.

As used herein, the terms "in combination" or "co-administration" can be used interchangeably to refer to the use of more than one therapy (e.g., one or more prophylactic and/or therapeutic agents). The use of the terms does not restrict the order in which therapies (e.g., prophylactic and/or therapeutic agents) are administered to a subject.

In some embodiments, the methods can include administering simultaneously, separately, or sequentially, a therapeutically effective amount of one or more proton pump inhibitors.

In other embodiments, the methods can include administering simultaneously, separately or sequentially, a therapeutically effective amount of one or more acid pump antagonists.

In other embodiments, the methods can include administering simultaneously, separately, or sequentially one or more agents chosen from an antacid, a histamine H2-receptor antagonist, a γ-aminobutyric acid-β (GABA-B) agonist, a prodrug of a GABA-B agonist, and a protease inhibitor.

When co-administered with other agents, an "effective amount" of the second agent will depend on the type of drug used. Suitable dosages are known for approved agents and can be adjusted by the skilled artisan according to the condition of the subject, the type of condition(s) being treated and the amount of a compound described herein being used. In cases where no amount is expressly noted, an effective amount should be assumed. For example, compounds described herein can be administered to a subject in a dosage range from between about 0.01 to about 10,000 mg/kg body weight/day, about 0.01 to about 5000 mg/kg body weight/day, about 0.01 to about 3000 mg/kg body weight/day, about 0.01 to about 1000 mg/kg body weight/day, about 0.01 to about 500 mg/kg body weight/day, about 0.01 to about 300 mg/kg body weight/day, about 0.01 to about 100 mg/kg body weight/day.

While the two or more agents in the combination therapy can be administered simultaneously, they need not be. For example, administration of a first agent (or combination of agents) can precede administration of a second agent (or combination of agents) by minutes, hours, days, or weeks. Thus, the two or more agents can be administered within minutes of each other or within 1, 2, 3, 6, 9, 12, 15, 18, or 24 hours of each other or within 1, 2, 3, 4, 5, 6, 7, 8, 9, 10, 12, or 14 days of each other or within 2, 3, 4, 5, 6, 7, 8, 9, or 10 weeks of each other. In some cases even longer intervals are possible. While in many cases it is desirable that the two or more agents used in a combination therapy be present in within the patient's body at the same time, this need not be so.

Combination therapy can also include two or more administrations of one or more of the agents used in the combination. For example, if agent X and agent Y are used in a combination, one could administer them sequentially in any combination one or more times, e.g., in the order X-Y-X, X-X-Y, Y-X-Y, Y-Y-X, X-X-Y-Y, etc.

The present disclosure also relates to the gastro-retentive, oral dosage form described herein for use in treating or preventing an upper gastrointestinal tract disorder or throat disorder, which is particularly useful as a first-line or initial therapy, comprising administering to a patient in need thereof a combination therapeutic regimen as described in the kits and dosage forms provided and discussed herein. "First-line" or "initial" treatment refers to treatment in the first instance after a new diagnosis of an upper gastrointestinal tract disorder, or after a relapse of an upper gastrointestinal tract disorder following cessation of treatment. However, the treatment can be useful in any upper gastrointestinal tract disorder patient who is not responding to monotherapy with PPIs or bile acid sequestrants.

PPI drugs are substituted benzimidazole compounds that specifically inhibit gastric acid secretion by affecting the H⁺/K⁺ ATPase enzyme system (the proton pump). These drugs, for example esomeprazole, are rapidly absorbed and have very short half-lives. However, they exhibit prolonged binding to the H⁺/K⁺ ATPase enzyme. The anti-secretory effect reaches a maximum in about 4 days with once-daily dosing. Because of these characteristics, patients beginning PPI therapy do not receive maximum benefit of the drug and healing may not begin for up to 5 days after therapy begins when PPIs are used alone for initial therapy of upper GI tract disorders.

Proton pump inhibitors (PPIs) are potent inhibitors of gastric acid secretion, inhibiting H⁺/K⁺ ATPase, the enzyme involved in the final step of hydrogen ion production in the parietal cells. The term proton pump inhibitor includes, but is not limited to, omeprazole (as sold under the brand-names PRILOSEC^{®}, LOSEC^{®}, or ZEGERID^{®}), lansoprazole (as sold under the brand-name PREVACID®, ZOTON®, or INHIBITOL®), rabeprazole (as sold under the brand-name RABECID®, ACIPHEX®, or PARIET®), pantoprazole (as sold under the brand-name PROTONIX®, PROTIUM®, SOMAC®, or PANTOLOC®), tenatoprazole (also referred to as benatoprazole), and leminoprazole, including isomers, enantiomers and tautomers thereof (e.g., esomeprazole (as sold under the brand-name NEXIUM®)), Dexlansoprazole, Dexrabeprazole, (S)-Pantoprazole, Ilaprazole and alkaline salts thereof; The following patents describe various benzimidazole compounds suitable for use in the disclosure described herein: U.S. Pat. No. 4,045,563, U.S. Pat. No. 4,255,431, U.S. Pat. No. 4,359,465, U.S. Pat. No. 4,472,409, U.S. Pat. No. 4,508,905, JP-A-59181277, U.S. Pat. No. 4,628,098, U.S. Pat. No. 4,738,975, U.S. Pat. No. 5,045,321, U.S. Pat. No. 4,786,505, U.S. Pat. No. 4,853,230, U.S. Pat. No. 5,045,552, EP-A-295603, U.S. Pat. No. 5,312,824, EP-A-166287, U.S. Pat. No. 5,877,192, EP-A-519365, EP5129, EP 174,726, EP 166,287 and GB 2,163,747. Thus, proton pump inhibitors and their pharmaceutically acceptable salts, which are used in accordance with the present disclosure, are known compounds and can be produced by known processes. In certain embodiments, the proton pump inhibitor is omeprazole, either in racemic mixture or only the (-)enantiomer of omeprazole (i.e. esomeprazole), as set forth in U.S. Pat. No. 5,877,192.

Omeprazole is typically administered in a 20 mg dose/day for active duodenal ulcer for 4-8 weeks; in a 20 mg dose/day for gastro-esophageal reflux disease (GERD) or severe erosive esophagitis for 4-8 weeks; in a 20 mg dose/twice a day for treatment of Helicobacter pylori (in combination with other agents); in a 60 mg dose/day for active duodenal ulcer for 4-8 weeks and up to 120 mg three times/day, and in a 40 mg dose/day for gastric ulcer for 4-8 weeks. Such dosages are contemplated to be within the scope of the present disclosure. Thus, in certain embodiments of the present disclosure, the amount of proton pump inhibitor which is included in the dosage form is an amount which is considered to be therapeutically effective, in accordance with the dosages set forth above for a variety of disease states. In other embodiments of the present disclosure, the dose of proton pump inhibitor is sub-therapeutic. For example, when the drug is omeprazole, the dosage form may contain from about 0.1 mg to about 120 mg omeprazole.

Lansoprazole is typically administered about 15-30 mg/day; rabeprazole is typically administered 20 mg/day and pantoprazole is typically administered 40 mg/day. However, any therapeutic or sub-therapeutic dose of these agents is considered within the scope of the present disclosure.

Acid pump antagonists (APAs) acting by K(+)-competitive and reversible (as opposed to irreversible PPIs) binding to the gastric proton pump, which is the final step for activation of acid secretion in the parietal cell. One class of APAs are imidazopyridines. BY841 was selected from this class and is chemically a (8-(2-methoxycarbonylamino-6-methyl-phenylmethylamino )-2,3-dimethyl-imidazo [1,2-a]-pyridine). In pharmacological experiments such as pH-metry in the conscious, pentagastrin-stimulated fistula dog, BY841 proved to be superior to both ranitidine and omeprazole by rapidly elevating intragastric pH up to a value of 6. The duration of this pH elevation in the dog was dose-dependent. Using both acid output and continuous 24-hr pH measurements, a pronounced antisecretory effect of BY841 has been found. Actually, a single 50 mg oral dose of BY841 immediately elevated intragastric pH to about 6. Higher doses caused a dose-dependent increase in duration of the pH-elevation, without any further increase in maximum pH values. Twice daily administration was more effective than once a day administration of the same daily dose. With both regimens, the duration of the pH-elevating effect of BY841 further increased upon repeated daily administration. This demonstrates lack of tolerance development, the latter being a well-known disadvantage of H2-receptor antagonists. In comparison with the standard dose of omeprazole, BY841 administered at a dose of 50 mg or 100 mg twice daily is markedly more effective on Day one of treatment, and both doses are at least as potent as omeprazole following repeated daily administration.

Examples of some APAs that could be used in the treatment of the invention include, but are not limited to: BY-841 (Prumaprazole), Sch-28080, YJA-20379-8, YJA-20379-1, SPI-447, SK&F-97574, AU-2064, SK&F-96356, T-330, SK&F-96067, SB-641257A (YH-1885, Revaprazan hydrochloride, Revanex^{R}), CS-526, R-105266, Linaprazan, Sorapraza, DBM-819, KR-60436, RQ-00000004 (RQ-4) and YH-4808.

The oral dosage forms disclosed herein may also be administered in conjunction with, other agents for treating the gastrointestinal tract, such as histamine H₂ receptor blockers, motility agents (gastroprokinetics), antacids, antiulcerative agents, γ-aminobutyric acid-β (GABA-B) agonists, prodrugs of GABA-B agonists, GCC agonists and/or protease inhibitors. Non-limiting examples of these additional agents include those selected from the group consisting of cinitapride, cisapride, fedotozine, loxiglumide, alexitol sodium, almagate, aluminum hydroxide, aluminum magnesium silicate, aluminum phosphate, azulene, basic aluminum carbonate gel, bismuth aluminate, bismuth phosphate, bismuth subgallate, bismuth subnitrate, calcium carbonate, dihydroxyaluminum aminoacetate, dihydroxyaluminum sodium carbonate, ebimar, magaldrate, magnesium carbonate hydroxide, magnesium hydroxide, magnesium oxide, magnesium peroxide, magnesium phosphate (tribasic), magnesium silicates, potassium citrate, sodium bicarbonate, aceglutamide aluminum complex, acetoxolone, aldioxa, arbaprostil, benexate hydrochloride, carbenoxolone, cetraxate, cimetidine, colloidal bismuth subcitrate, ebrotidine, ecabet, enprostil, esaprazole, famotidine, gefamate, guaiazulene, irsogladine, misoprostol, nizatidine, omoprostil, γ-Oryzanol, pifamine, pirenzepine, plaunotol, polaprezinc, ranitidine, rebamipide, rioprostil, rosaprostol, rotraxate, roxatidine acetate, sofalcone, spizofarone, sucralfate, telenzepine, teprenone, trimoprostil, trithiozine, troxipide, zolimidine, baclofen, R-baclofen, XP19986 (CAS Registry No. 847353-30-4), pepstatin and other pepsin inhibitors (e.g., sodium benzoate); and chymotrypsin and trypsin inhibitors. A wide variety of trypsin and chymotrypsin inhibitors are known to those skilled in the art and can be used in the methods described herein. Such trypsin and chymotrypsin inhibitors can include tissue-factor-pathway inhibitor; α-2 antiplasmin; serpin α-1 antichymotrypsin family members; gelin; hirustasin; eglins including eglin C; inhibitors from *Bombyx mori* (see; e.g.; JP 4013698 A2 and JP 04013697 A2; CA registry No. 142628-93-1); hirudin and variants thereof; secretory leukocyte protease inhibitor (SLPI); α-1 anti-trypsin; Bowman-Birk protease inhibitors (BBIs); chymotrypsin inhibitors represented by CAS registry Nos. 306762-66-3, 306762-67-4, 306762-68-5, 306762-69-6, 306762-70-9, 306762-71-0, 306762-72-1, 306762-73-2, 306762-74-3, 306762-75-4, 178330-92-2, 178330-93-3, 178330-94-4, 81459-62-3, 81459-79-2, 81460-01-7, 85476-59-1, 85476-62-6, 85476-63-7, 85476-67-1, 85476-70-6, 85858-66-8, 85858-68-0, 85858-69-1, 85858-70-4, 85858-71-5, 85858-72-6, 85858-73-7, 85858-75-9, 85858-77-1, 85858-79-3, 85858-81-7, 85858-83-9, 85858-84-0, 85858-85-1, 85858-87-3, 85858-89-5, 85858-90-8, 85858-92-0, 85879-03-4, 85879-05-6, 85879-06-7, 85879-08-9, 85858-74-8, 90186-24-6, 90185-93-6, 89703-10-6, 138320-33-9 (YS3025), 94149-41-4 (MR889), 85858-76-0, 89703-10-6, 90185-92-5, 90185-96-9, 90185-98-1, 90186-00-8, 90186-01-9, 90186-05-3, 90186-06-4, 90186-07-5, 90186-08-6, 90186-09-7, 90186-10-0, 90186-11-1, 90186-12-2, 90186-13-3, 90186-14-4, 90186-22-4, 90186-23-5, 90186-24-6, 90186-25-7, 90186-27-9, 90186-28-0, 90186-29-1, 90186-31-5, 90186-35-9, 90186-43-9, 90209-88-4, 90209-89-5, 90209-92-0, 90209-94-2, 90209-96-4, 90209-97-5, 90210-01-8, 90210-03-0, 90210-04-1, 90210-25-6, 90210-26-7, 90210-28-9, 90230-84-5, 90409-84-0, 95460-86-9, 95460-87-0, 95460-88-1, 95460-89-2, 95460-91-6, 114949-00-7, 114949-01-8, 114949-02-9, 114949-03-0, 114949-04-1, 114949-05-2, 114949-06-3, 114949-18-7, 114949-19-8, 114964-69-1, 114964-70-4, 9076-44-2 (chymostatin), 30827-99-7 (Pefabloc), 618-39-3 (benzamidine), 80449-31-6 (urinistatin), 130982-43-3, 197913-52-3, 179324-22-2, 274901-16-5, 792163-40-7, 339169-59-4, 243462-36-4, 654671-78-0, 55123-66-5 (leupeptin), 901-47-3, 4272-74-6, 51050-59-0, 221051-66-7, 80449-31-6, 55-91-4, 60-32-2, 88070-98-8, 87928-05-0, 402-71-1 (benzenesulfonamide), 139466-47-0, CI-2A (see US5167483), CI-2A (seebWO9205239), WCI-3 (see Shibata et al. 1988 J Biochem (Tokyo) 104:537-43), WCI-2 (see Habu et al. 1992 J Biochem (Tokyo) 111:249-58), and WCI-x (Habu et al., supra) and 178330-95-5; and compounds with chymotrypsin inhibition activity described in patent publications JP 56092217 A2, US4755383, US4755383, US4639435, US4620005, US4898876, and EP0128007.

Examples of other therapeutic agents that may be combined with a compound of this disclosure, either administered separately or in the same pharmaceutical composition, include, but are not limited to linaclotide, IW-9179, plecanatide and SP-333

### Kits

Also disclosed herein are kits for treating an upper GI tract or throat disorder comprising, in one or more containers, a therapeutically effective amount of a bile acid sequestrant in the form of a gastro-retentive sustained-release dosage forms described herein, and a label or packaging insert containing instructions for use.

The compounds and pharmaceutical formulations described herein may be contained in a kit. The kit may include single or multiple doses of two one or more agents, each packaged or formulated individually, or single or multiple doses of two or more agents packaged or formulated in combination. Thus, one or more agents can be present in first container, and the kit can optionally include one or more agents in a second container. The container or containers are placed within a package, and the package can optionally include administration or dosage instructions. A kit can include additional components such as syringes or other means for administering the agents as well as diluents or other means for formulation. Thus, the kits can comprise: a) a dosage form described herein (one or more than one units to make up the necessary therapeutic dosage); and b) a container or packaging. The kits may optionally comprise instructions describing a method of using the pharmaceutical compositions in one or more of the methods described herein (e.g. preventing or treating one or more of the diseases and disorders described herein). The kit may optionally comprise a second pharmaceutical composition comprising one or more additional agents described herein for co therapy use, a pharmaceutically acceptable carrier, vehicle or diluent.

A kit includes a container or packaging for containing the pharmaceutical compositions and may also include divided containers such as a divided bottle or a divided foil packet. The container can be, for example a paper or cardboard box, a glass or plastic bottle or jar, a re-sealable bag (for example, to hold a "refill" of tablets for placement into a different container), or a blister pack with individual doses for pressing out of the pack according to a therapeutic schedule. It is feasible that more than one container can be used together in a single package to market a single dosage form. For example, tablets may be contained in a bottle which is in turn contained within a box.

An example of a kit is a so-called blister pack. Blister packs are well known in the packaging industry and are being widely used for the packaging of pharmaceutical unit dosage forms (tablets, capsules, and the like). Blister packs generally consist of a sheet of relatively stiff material covered with a foil of a preferably transparent plastic material. During the packaging process, recesses are formed in the plastic foil. The recesses have the size and shape of individual tablets or capsules to be packed or may have the size and shape to accommodate multiple tablets and/or capsules to be packed. Next, the tablets or capsules are placed in the recesses accordingly and the sheet of relatively stiff material is sealed against the plastic foil at the face of the foil which is opposite from the direction in which the recesses were formed. As a result, the tablets or capsules are individually sealed or collectively sealed, as desired, in the recesses between the plastic foil and the sheet. Preferably the strength of the sheet is such that the tablets or capsules can be removed from the blister pack by manually applying pressure on the recesses whereby an opening is formed in the sheet at the place of the recess. The tablet or capsule can then be removed via said opening.

It may be desirable to provide written memory aid containing information and/or instructions for the physician, pharmacist or subject regarding when the medication is to be taken. A "daily dose" can be a single tablet or several tablets to be taken on a given day. When the kit contains separate compositions, a daily dose of one or more compositions of the kit can consist of one tablet or capsule while a daily dose of another or more compositions of the kit can consist of several tablets or capsules. A kit can take the form of a dispenser designed to dispense the daily doses one at a time in the order of their intended use. The dispenser can be equipped with a memory-aid, so as to further facilitate compliance with the regimen. An example of such a memory-aid is a mechanical counter which indicates the number of daily doses that have been dispensed. Another example of such a memory-aid is a battery-powered micro-chip memory coupled with a liquid crystal readout, or audible reminder signal which, for example, reads out the date that the last daily dose has been taken and/or reminds one when the next dose is to be taken.

These and other objects, features and advantages of this disclosure will become apparent from the following detailed description of the various aspects of the disclosure taken in conjunction with the accompanying Examples.

### EXAMPLES

As used herein, all abbreviations, symbols and conventions are consistent with those used in the contemporary scientific literature. See, e.g. Janet S. Dodd, ed., The ACS Style Guide: A Manual for Authors and Editors, 2nd Ed., Washington, D.C.: American Chemical Society, 1997.

### Example 1: Procedure for the manufacture of large scale batches

### 1. Intra-granular Blending

Intragranular components (except magnesium stearate) were dispensed and passed through a 20-mesh screen. Components were added to a V-blender and blended for 10 minutes at 25 RPM. Intragranular magnesium stearate was then dispensed and passed through a 20-mesh screen. Magnesium stearate was added to the V-blender and blended for 2 minutes at 25 RPM.

The resulting intragranular blend was discharged from the V-blender.

### 2. Slugging

A 24-station Fette tablet press was equipped with eight 0.6250" round flat, plain-faced type B tooling elements. The intragranular blend obtained above was added to the hopper of the tablet press. Using a tablet press turret speed of 15-25 RPM, the blend was compressed into slugs with a target weight of 1200-1400 mg and a hardness of 9-15 kp. In-process checks were performed during slugging for appearance, weight, hardness, and thickness.

### 3. Milling

A Quadro Comil apparatus was equipped with either of two screens (2C125G03723390 - hole size of 0.125" or 2C 109G03727333 - hole size of 0.109") and a flat (square) impeller. The slugs obtained above were transferred to the hopper of the Quadro Comil apparatus. The slugs were milled at 2000 RPM and the milled granulation recovered. The yield for the recovered granulation was calculated.

### 4. Extra-granular Blending

The necessary amounts of the extragranular components were determined based on the yield of the milled granulation step. The extragranular components (except magnesium stearate) were then dispensed and passed through a 20-mesh screen. A portion of the milled granulation was added to a V-blender. The dispensed extragranular components were then added to the V-blender and blended for 10 minutes at 25 RPM. The extragranular magnesium stearate was then dispensed and passed through a 20-mesh screen. The extragranular magnesium stearate was added to the V-blender and blended for 2 minutes at 25 RPM. The dry blend was discharged from the V-blender.

### 5. Tableting

A 24-station Fette tablet press was equipped with eight 0.4724" x 0.7480" modified oval, concave, plain-faced type B tooling elements. The dry blend was added to the hopper of the tablet press. Using a tablet press turret speed of 15-25 RPM, the dry blend was compressed into tablets with a target weight of 950-1050 mg and a hardness of 9-15 kp. In-process checks were performed during tableting for appearance, weight, hardness, and thickness. The tablets obtained were stored in double-lined poly bags with desiccant packs in between the bags at 2-8 °C until coating was carried out.

### 6. Coating

The coating solution - 5% total solids concentration with a ratio by weight of 3:1 HPMC (Grade - E50 Premium LV): diacetylated monoglycerides, NF Grade-(Myvacet 9-45K). The solution was prepared by dispensing the diacetylated monoglycerides into sterile water for irrigation, USP. Then HPMC (E50) was slowly added to the solution while stirring and stirring was continued until a homogeneous suspension was produced.

Coating was then performed on a Vector-Freund LDCS Hi-Coater apparatus with the following settings:
- Inlet temperature: 65°C (Range: 60-70°C)
- Airflow: 60 CFM (Range: 55-65 CFM)
- Spray rate: 10 g/min (Range: 6-14 g/min)
- Atomization air pressure: 20 psi (Range: 15-25 psi)
- Pattern air pressure: 13 psi (Range: 10-16 psi)
- Exhaust temperature (output result dependent on other coater settings): target = 43°C

The tablets were added to the appropriate size coating pan and the pan installed in the coater. The spray nozzle was then installed and the atomization and pattern air pressures were set. The inlet and exhaust air fans were turned on and the inlet temperature was set to the target value. The pan was then set to a slow jog speed and cycle while the exhaust temperature increased to the target value. The inlet airflow was adjusted to the target flow rate. Once the exhaust temperature had reached the target value, a sample of tablets was retrieved from the coating pan and their weight obtained. The target weight for a weight gain of 5-7.5% was calculated based on the weight of the sample. The sample was placed back in the coating pan and the pan was immediately set to a target speed of 16 RPM (range: 12-20 RPM). Immediately, spraying of the coating solution at the target spray rate was started. At different intervals during the coating process, a sample of tablets was obtained from the coating pan and the weight was measured. Once the target weight gain of 5-7.5% had been obtained for a sample (the weight gain for the batches that have been produced to date is shown in the table below), the heating of the inlet airflow was turned off and the pan returned to the previously used jog conditions. Once the exhaust temperature reached a value of < or = to 30°C, the jog of the pan was stopped and the tablets retrieved.

### Example 2: large scale batches manufactured

The following large scale batches were manufactured following the procedure described in Example 1. Table 1 below summarizes the excipient amounts used in the manufacture of each of the 6 batches.

**Table 1**

| **%** | **Previous Batches*** | **Batch 1** | **Batch 2** | **Batch 3** | **Batch 4** | **Batch 5** |
|---|---|---|---|---|---|---|
| **Intragranular** | | | | | | |
| Colesevelam HCl | 50 | 50 | 50 | 50 | 50 | 50 |
| Prosolv SMCC90 | 9 | | 9 | | 9 | |
| KG-1000 | | 9 | | 9 | | 9 |
| Mg Stearate | 0.25 | 0.25 | 0.25 | 0.25 | 0.25 | 0.25 |

| **Extragranular** | | | | | | |
|---|---|---|---|---|---|---|
| Prosolv SMCC90 | 10.5 | | 15.5 | | 10.5 | |
| KG-1000 | | 10.5 | | 15.5 | | 10.5 |
| PEO N60K | 25 | 25 | 25 | 25 | 25 | 25 |
| HPMCE5 | 5 | 5 | | | | |
| Trehalose P | | | | | 5 | 5 |
| Mg Stearate | 0.25 | 0.25 | 0.25 | 0.25 | 0.25 | 0.25 |

| **Coating (prepared at 5 wt% solids in water)** | | | | | | |
|---|---|---|---|---|---|---|
| HPMC E50 | 75 | 75 | 75 | 75 | 75 | 75 |
| Myvacet 9-45K | 25 | 25 | 25 | 25 | 25 | 25 |

Microcrystalline cellulose (MCC) is used as a filler/compression aid in the formulation. Two types of MCC have been evaluated. Prosolv SMCC90, silicified microcrystalline cellulose has been demonstrated by one of the manufacturers to have improved powder flow over non-silicified MCC.

KG-1000 is used as an alternative to SMCC90, another type of MCC. According to one of the manufacturers: KG-1000 has the lowest bulk density among MCC grades. KG-1000 shows superior compactability compared with other standard MCC grades. KG-1000 particles have extremely large L/D value. The particles easily arrange perpendicularly to the applied force upon compaction; therefore the contact area of the MCC particles is increased. Entanglement of particles also easily occurred under compression force which provides additional compactability.

Mg Stearate is used as a lubricant in the formulation.

Trehalose is used as a binder and drug release aid.

PEO (Polyethylene oxide of different molecular weights) is used as the controlled release, swelling and erodible polymer that imparts gastro-retentive properties to the tablet.

HPMC (hydroxypropylmethylcellulose, hypromellose) is an erodible hydrophilic polymer and is used as a binder in the formulation.

Myvacet 9-45K is a type of diacetylated monoglyceride and is used as a plasticizer in the coating of the formulation.

### Example 3A: In-vitro dissolution/drug release

Drug release rates for the six formulations produced on large scale were measured using an indirect method, given that colesevelam and colesevelam hydrochloride are insoluble polymers and their concentrations cannot be measured by the standard direct HPLC methods used for soluble drugs.

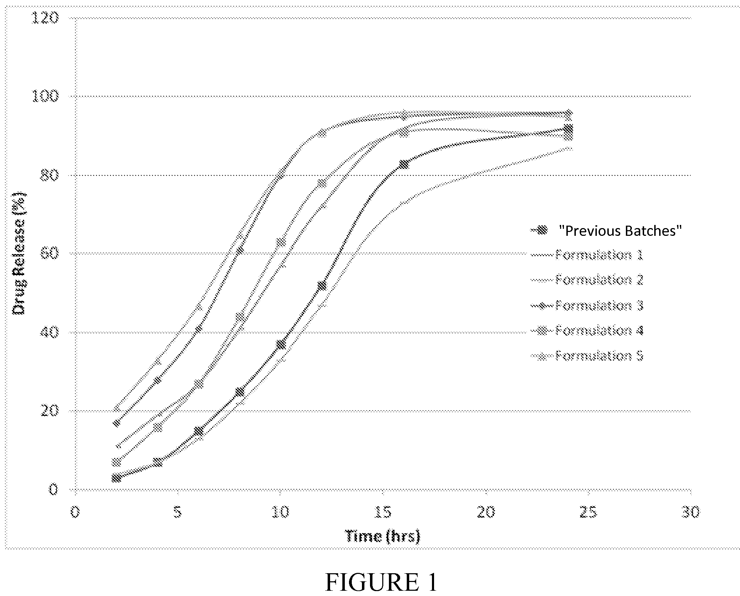

The drug release rate of the tablets was measured *in vitro* in acetate buffer pH 4.5 (100 mM) containing 2 mg/mL concentration of a known bile acid (glycocholic acid). The depletion of bile acid during the swelling and erosion of the tablets was measured and compared with the values obtained for a series of standard solutions of known bile acid concentration. The rate of depletion of the bile acids corresponds to the drug release rate. These drug release rate results were obtained using a USP Type II (paddle) apparatus with the tablets placed in sinkers. Results are summarized in **Figure 1****.**

### Example 3B: In vitro-disintegration

Disintegration media (800 mL of pH 4.5, 100 mM acetate buffer) was placed into three disintegration vessels and allowed to heat up to approximately 37°C. A small droplet of media was placed on the edge of disintegration discs. These discs were then placed on tablets and held together for about 5-10 seconds. The tablet and disc were placed on a disintegration tester and the test was started. Three tablets were tested simultaneously in each vessel with visual observation of the disintegration time. Under these conditions, Batch 3 disintegrated in about 8.5 - 9 hrs. Under these conditions, Batch 4 disintegrated in about 9.5 - 10 hrs.

### Example 3C: In vitro-swellability

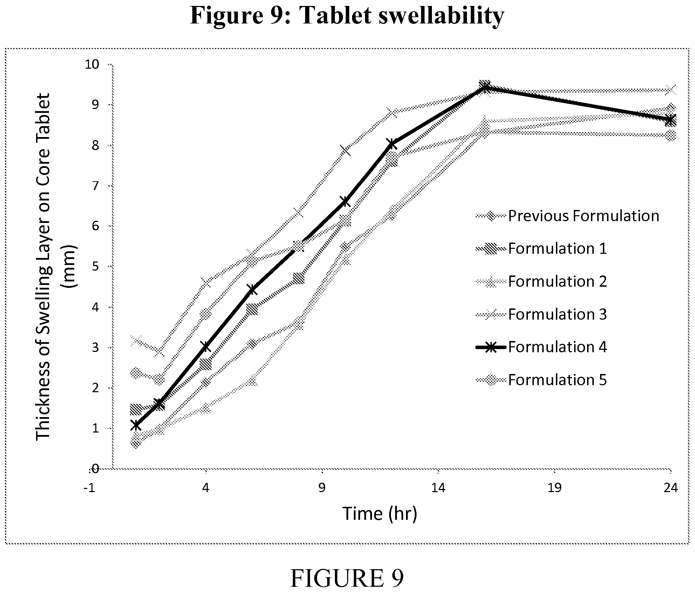

The swellability assessments were performed by adhering tablets to glass microscope slides then placing the slides into 1 L beakers. A volume of 900 mL of deionized water was then carefully added to each beaker. One beaker was used for each time-point and the beakers were placed on an orbital shaker at a temperature of 37°C and 100 RPM. For each time-point, a beaker was removed from the shaker and the tablet adhered to the slide was retrieved. The tablet with the slide was then placed on a TA.xt plus Texture Analyzer (from Texture Technologies Co.) equipped with a cylindrical probe. The probe was lowered to be directly over the tablet. The probe was then lowered further into the swelled layer of the tablet until it reached the unswelled core of the tablet. The measurement of force as a function of distance was obtained as the probe penetrated the tablet. The distance from the beginning of the swelled layer until the core of the tablet represents the thickness of the swelled layer. The measurements obtained on separate tablets at different time points can be plotted to determine the swelling rate of the tablets. See Figure 9 for some data obtained with the formulations (batches 1 to 5) prepared herein.

### Examples 4: Identification of manufacturing parameters that influence drug release rates

Certain manufacturing parameters were identified to play a large role in the drug release rate in addition to the formulation composition parameters. These included coating level and particle size of the granulation.

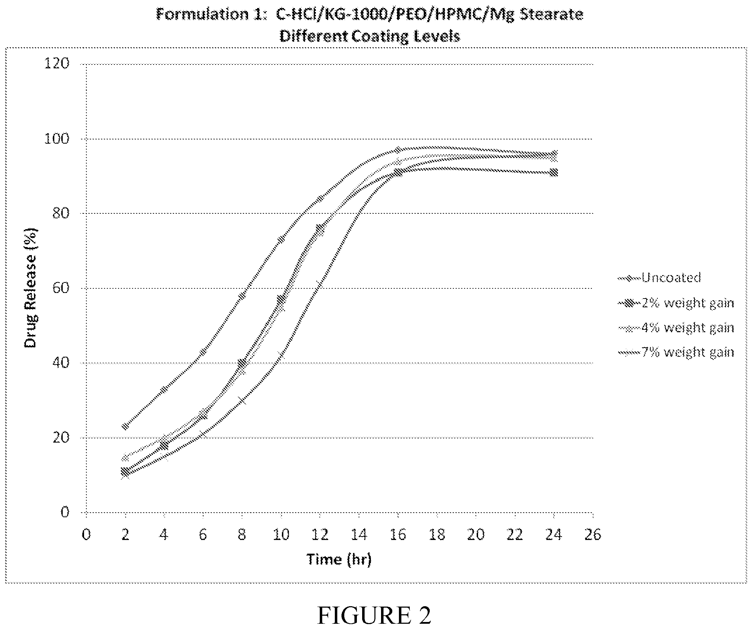

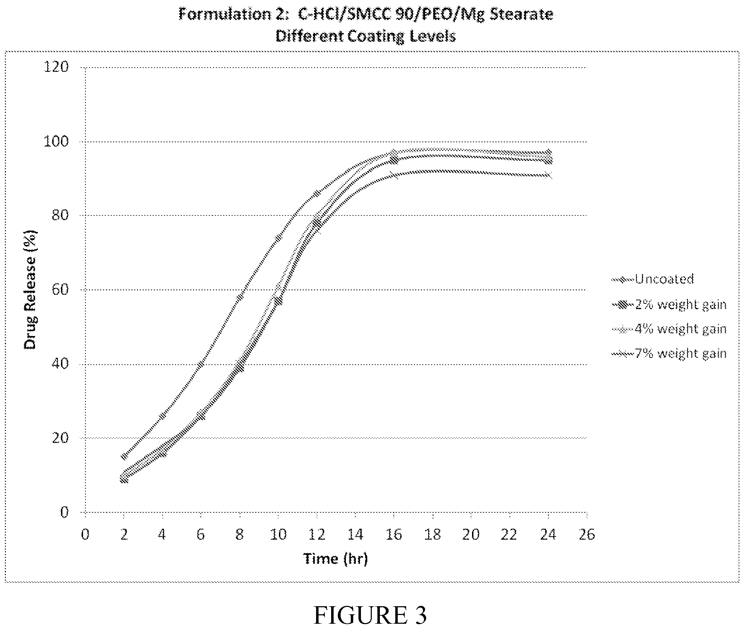

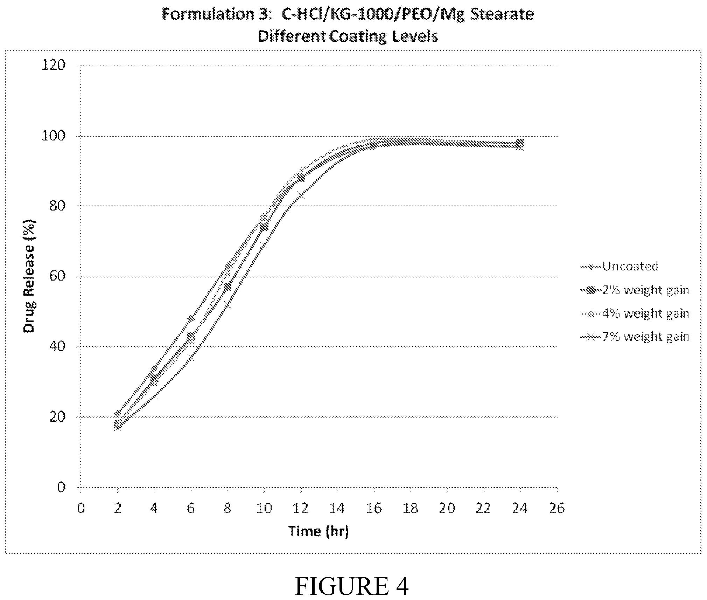

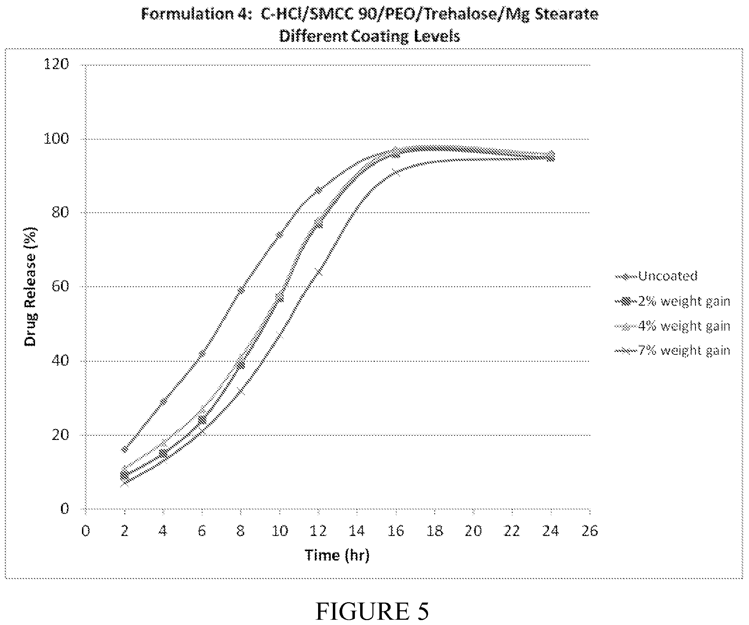

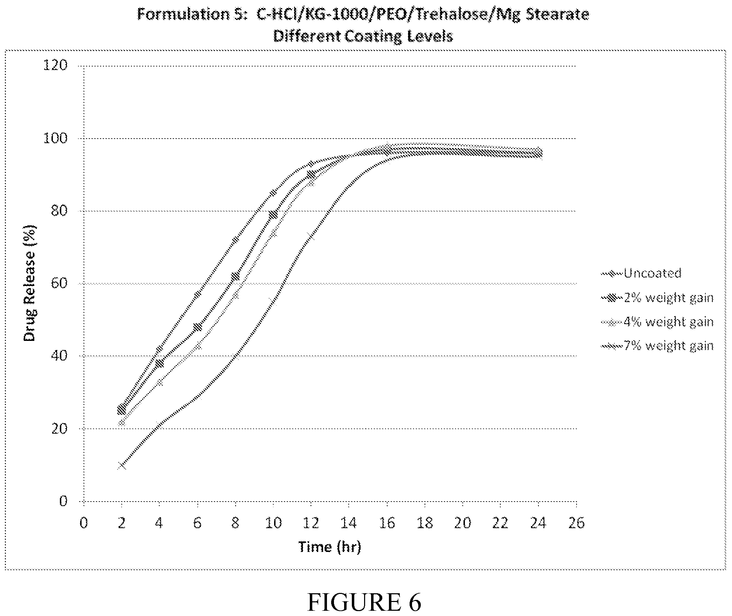

The coating level impact on drug release rates for Formulations 1-5 (tablets coated at levels of approximately 2.5, 4.5, and 7% weight gains compared to uncoated tablets) are summarized in the graphs depicted in **Figures 2 through 6****.**

### Example 5: Alternative protocol for preparation of Formulation 3

Formulation 3 was re-synthesized using an alternative large scale protocol described below:

### 1. Intra-granular blending

Intra-granular components (except magnesium stearate) were dispensed and passed through a 20-mesh screen. Components were added to a V-blender and blended for 10 minutes at 25 RPM. Intra-granular magnesium stearate was then dispensed and passed through a 20-mesh screen. Magnesium stearate was added to the V-blender and blended for 3 minutes at 25 RPM. The resulting intra-granular blend was discharged from the V-blender.

### 1. Slugging

A 24-station Fette tablet press was equipped with eight 0.6250" round flat, plain-faced type B tooling elements. The intra-granular blend obtained above was added to the hopper of the tablet press. Using a tablet press turret speed of 15-25 RPM, the blend was compressed into slugs with a target weight of 1200-1400 mg and a hardness of 9 - 15 kp. In-process checks were performed during slugging for appearance, weight, hardness, and thickness.

### 2. Milling

A Quadro Comil apparatus was equipped with either of two screens (2CI25G03723390 - hole size of 0.125" or 2C109G03727333 - hole size of 0.109") and a flat (square) impeller. The slugs obtained above were transferred to the hopper of the Quadro Comil apparatus. The slugs were milled at 2000 RPM and the milled granulation recovered. The yield for the recovered granulation was calculated.

### 3. Extra-granular blending

The necessary amounts of the extra-granular components were determined based on the yield of the milled granulation step. The extra-granular components (except magnesium stearate) were then dispensed and passed through a 20-mesh screen. A portion of the milled granulation was added to a V-blender. The dispensed extra-granular components were then added to the V-blender and blended for 10 minutes at 19 RPM. The extra-granular magnesium stearate was then dispensed and passed through a 20-mesh screen. The extra-granular magnesium stearate was added to the V-blender and blended for 3 minutes at 19 RPM. The dry blend was discharged from the V-blender.

### 4. Tableting

A 24-station Fette tablet press was equipped with eight 0.4724" x 0.7480" modified oval, concave, plain-faced type B tooling elements. The dry blend was added to the hopper of the tablet press. Using a tablet press turret speed of 15-25 RPM, the dry blend was compressed into tablets with a target weight of slugs with a target weight of 950-1150 mg and a hardness of 12 - 30 kp. In-process checks were performed during tableting for appearance, weight, hardness, and thickness. The tablets obtained were stored in double-lined poly bags with desiccant packs in between the bags at 2-8°C until coating was carried out.

### 6. Coating

The coating solution - 5% total solids concentration with a ratio by weight of 3:1 HPMC (Grade - E50 Premium LV) : diacetylated monoglycerides, NF Grade (Myvacet 9-45K). The solution was prepared by dispensing the diacetylated monoglycerides into sterile water for irrigation, USP. Then HPMC (E50) was slowly added to the solution while stirring and stirring was continued until a homogeneous suspension was produced. Coating was then performed in a Vector-Freund LDCS Hi-Coater apparatus with the following settings:
- Inlet temperature: 65°C (Range: 60-70°C)
- Airflow: 60 CFM (Range: 55-65 CFM)
- Spray rate: 12 g/min (Range: 6-14 g/min)
- Atomization air pressure: 20 psi (Range: 15-25 psi)
- Pattern air pressure: 13 psi (Range: 10-16 psi)
- Exhaust temperature (output result dependent on other coater settings): Target 45°C

The tablets were added to the appropriate size coating pan and the pan installed in the coater. The spray nozzle was then installed and the atomization and pattern air pressures were set. The inlet and exhaust air fans were turned on and the inlet temperature was set to the target value. The pan was then set to a slow jog speed and cycle while the exhaust temperature increased to the target value. The inlet airflow was adjusted to the target flow rate. Once the exhaust temperature had reached the target value, a sample of tablets was retrieved from the coating pan and their weight obtained. The target amount of coating solids to be sprayed onto the tablets to achieve a tablet weight gain of at least 3.0% from the coating solids was calculated based on the weight of the sample. The sample was placed back in the coating pan and the pan was immediately set to a target speed of 16 RPM (range: 12-20 RPM). Immediately, spraying of the coating solution at the target spray rate was started. At different intervals during the coating process, a sample of tablets was obtained from the coating pan and the weight was measured. Once the target weight gain based on the solids was at least 3.0% for a sample, the temperature of the inlet airflow was reduced to 50°C (range: 45-55°C). The pan speed was reduced to 3 RPM (range: 2-4 RPM) and the tablets were dried for 10 minutes in jog mode. The heating was then turned off and the pan returned to the previously used jog conditions. Once the exhaust temperature reached a value of < or = to 30°C, the jog of the pan was stopped and the tablets retrieved.

### Example 6: Dog imaging study

Beagle dogs were selected for use in this study based on anatomical, physiological, and biochemical similarities to humans, which facilitates the extrapolation of observed properties to humans. The stomach pylori of the dog is much smaller than that of a human, so this preclinical imaging data in dogs provides an *in vivo* assessment of the erosion time of the formulations but it does not provide an *absolute* gastric retention time in humans. However, comparison with an immediate release formulation suggests the tested formulations stay in the animal's stomach for extended periods of time.

In this study, dogs were administered a single tablet containing colesevelam hydrochloride formulated as a gastro-retentive formulation and containing barium sulfate strands in the shape of an "X". Following administration, the stomach was imaged using fluoroscopy and digital x-ray. Several formulations were tested in each dog, so that each formulation was administered once followed by at least a 2 day wash-out period prior to administering the next formulation until all animals were administered each formulation. Fluoroscopic images of the stomach and intestinal tract were taken at certain time points after dose administration until separation of the radiopaque "X" in the tablet was noted.

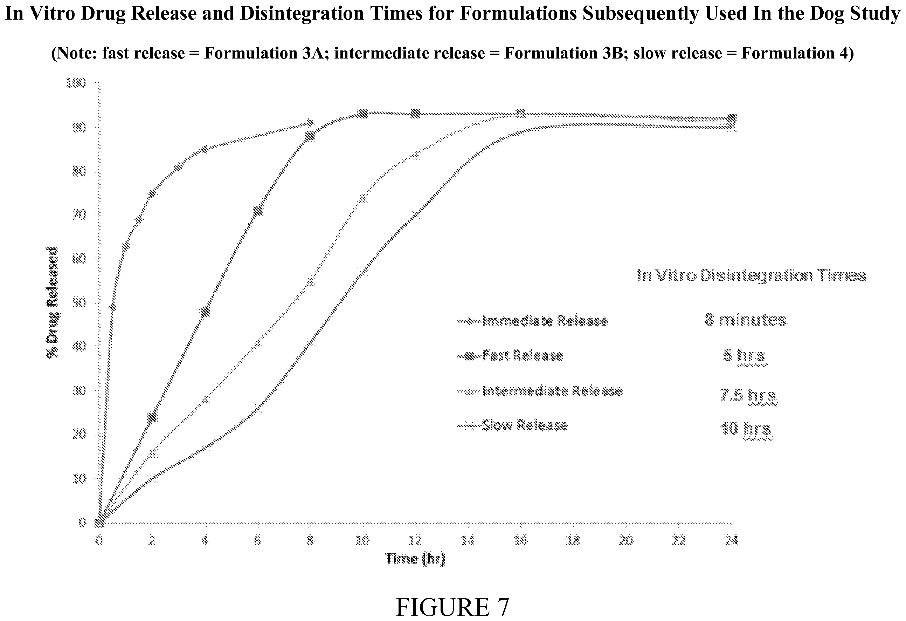

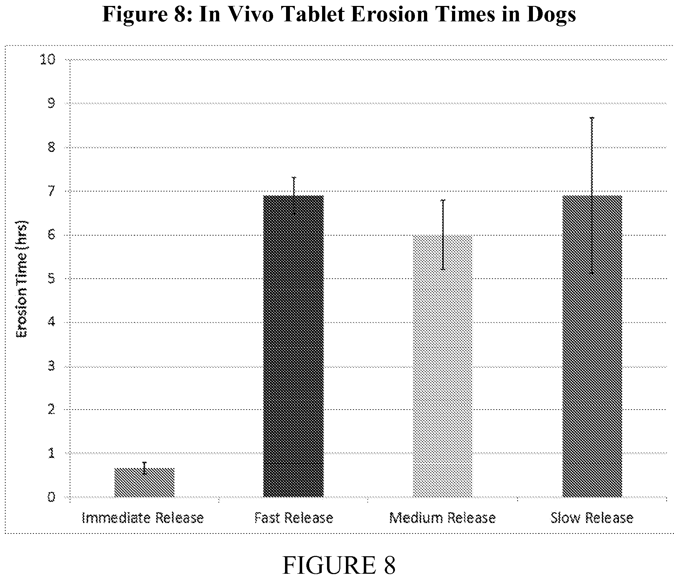

Four different formulations were analyzed in this study. A slow release formulation corresponding to Formulation 4 (or Batch 4) in the above protocols and Figures; an intermediate release formulation corresponding to Formulation 3 (or Batch 3) in the above protocols and Figures; a fast release formulation, which was obtained by the procedure described below; and an immediate release formulation, prepared also by the protocol described below. This study showed that the amount of time after oral administration needed for the various formulations to dissolve/erode in the canine stomach was approximately the same for all the formulations tested, despite having different release profiles *in vitro.* Most importantly, however, it showed that the immediate release formulation had the fastest average erosion rate at around 45 min, being completely eroded in the dog at least 5 hours faster than any of the other formulations tested (these data are summarized in Figure 7 and Figure 8).

***Preparation of the fast release formulation*** Intra-granular components (colesevelam HCl and microcrystalline cellulose - KG1000), were weighed out and passed through a 20 mesh screen then added to a v-blender. The mixture was blended for 10 minutes. Magnesium stearate was then passed through a 20 mesh screen, weighed out and added to the mixture contained in the v-blender. The mixture was blended for an additional 2 minutes. A rotary tablet press was setup with 0.6250" round, flat stainless steel tooling. The blend was added to the hopper. Slugs were prepared at a target weight of 1350 mg and a hardness of 11-12 kp. The slugs were milled with the Quadro Comill (screen size 7L 109G03127^{∗} 2769, impeller speed = 2000 rpm) and the resulting dry granules were collected. The yield after granulation was determined. The dry granules were added to the v-blender. Extra-granular excipients (polyethylene oxide and microcrystalline cellulose - KG1000) were passed through a 20 mesh screen, added to a v-blender and blended for 10 minutes. Magnesium stearate was then passed through a #20 mesh screen, weighed out and added to the blend. The mixture was blended for an additional 2 minutes. The tablet press was set up with 0.4724" x 0.7480" modified oval tooling. Tablets were produced at a target weight of 1015-1025 mg and target hardness of 14-15 kp.

The tablets were coated to a weight gain of 6.2% in a pan coater using a 5% solids concentration with diacetylated monoglycerides (Myvacet 9-45K) and hydroxypropylmethyl cellulose (Methocel E50) and the following process conditions:
Inlet temperature: 60°C
Exhaust temperature: 42-43°C
Airflow: 40 CFM
Solution rate: 4.0 g/m
Atomizing air: 1.5 bar
Pattern air: 1.0 bar
Drum speed: 16rpm

### Preparation of Immediate release formulation:

### Composition:

Core tablet: 74/25 / 1 Colesevelam HCl/ Silicified Microcrystalline cellulose - grade: SMCC90) / Magnesium stearate Coating : Core tablets are coated to a 8-9% weight gain. Coating composition: 75 / 25 Hydroxypropylmethyl Cellulose (Methocel E50) / Diacetylated Monoglycerides (Myvacet 9-45K)

### Preparation:

Colesevelam HCl and microcrystalline cellulose - SMCC90 were weighed out and passed through a 20 mesh screen then added to a v-blender. The mixture was blended for 10 minutes. Magnesium stearate was then passed through a 20 mesh screen, weighed out and added to the mixture contained in the v-blender. The mixture was blended for an additional 2 minutes. A rotary tablet press was setup 0.4724" x 0.7480" modified oval tooling. Tablets were produced at a target weight of approximately 850 mg and target hardness of 17 kp.

The tablets were coated to a weight gain of 8.7% in a pan coater using a 5% solids concentration with diacetylated monoglycerides (Myvacet 9-45K) and hydroxypropylmethyl cellulose (Methocel E50) and the following process conditions:
Inlet temperature: 60°C; Exhaust temperature: 42-43°C
Airflow: 40 CFM
Solution rate: 4.0 g/m
Atomizing air: 1.5 bar
Pattern air: 1.0 bar
Drum speed: 16rpm

The tablets were coated to a weight gain of 8.7% in a pan coater using a 5% solids concentration with diacetylated monoglycerides (Myvacet 9-45K) and hydroxypropylmethyl cellulose (Methocel E50) and the following process conditions:
Inlet temperature: 60°C; Exhaust temperature: 42-43°C
Airflow: 40 CFM
Solution rate: 4.0 g/m
Atomizing air: 1.5 bar
Pattern air: 1.0 bar
Drum speed: 16rpm

## Claims

1. A gastro-retentive, oral dosage form for use in the treatment of a disease of the upper gastrointestinal tract or the throat, wherein said dosage form is in the form of a tablet comprising:
a. a bile acid sequestrant selected from colesevelam and colesevelam hydrochloride, and
b. dispersed in a polymeric matrix comprising poly(alkylene)oxide, for sustained release of the bile acid sequestrant to the stomach, wherein the polymeric matrix comprises a poly(alkylene)oxide hydrophilic polymer such that, upon imbibition of gastric fluid, said dosage form swells to a size sufficient to promote gastric retention for a period of time of 3 hours or longer and wherein the bile acid sequestrant is released from the dosage form through erosion of the polymeric matrix over an extended period of time of at least 3 hours wherein he rate of drug release from the gastro-retentive dosage form is measured in vitro in acetate buffer at pH 4.5, using a USP Type II (paddle) apparatus with the tablets placed in sinkers.

2. The dosage form of claim 1, wherein the rate of drug release measured *in vitro,* in acetate buffer at pH 4.5, using a USP Type II (paddle) apparatus with the tablets placed in sinkers is such that not more than 40 % percent of the drug has been released from the dosage form after 4 hours.

3. The dosage form of claim 1 or claim 2, wherein the rate of drug release measured *in vitro,* in acetate buffer at pH 4.5, using a USP Type II (paddle) apparatus with the tablets placed in sinkers is such that between 75-100% of the drug has been released from the dosage form after 16 hours.

4. The dosage form of any one of the preceding claims, wherein the rate of drug release measured *in vitro,* in acetate buffer at pH 4.5, using a USP Type II (paddle) apparatus with the tablets placed in sinkers is such that between 85-100% of the drug has been released from the dosage form after 16 hours.

5. The dosage form of any one of the preceding claims, wherein the dose of bile acid sequestrant is between 400 mg and 600 mg.

6. The dosage form of claim 6, wherein the dose of bile acid sequestrant is 500 mg.

7. The dosage form of any one of the preceding claims, wherein said poly(alkylene)oxide comprises a poly(ethylene)oxide.

8. The dosage form of claim 7, wherein the poly(ethylene)oxide is present in an amount ranging from 40 weight percent ratio to 75 weight percent ratio.

9. The dosage form of claim 7, wherein the poly(ethylene)oxide is present in an amount ranging from 40 weight percent ratio to 60 weight percent ratio.

10. The dosage form of claim 7, wherein the poly(ethylene)oxide is present in an amount ranging from 45 weight percent ratio to 55 weight percent ratio.

11. The dosage form of claim 7, wherein the poly(ethylene)oxide is present in an amount ranging from 40 weight percent ratio to 50 weight percent ratio.

12. The dosage form of any one of the preceding claims, further comprising a coating,
wherein the level of coating is between 5 % and 7.5 % (weight: weight) of the total tablet weight.

13. The dosage form of any one of the preceding claims, wherein said gastro-retentive oral dosage form swells to a size that is at least 110 % of the original size within 30 minutes.

14. The gastro-retentive oral dosage form of any one of claims 1 to 12, wherein said gastro-retentive oral dosage form swells to a size that is at least 140 % of the original size within 2 hours.

15. A pharmaceutical composition comprising the gastro-retentive oral dosage form of any one of claims 1 to 14 and an additional therapeutic agent.

16. The gastro-retentive, oral dosage form of any one of claims 1-14 or the pharmaceutical composition of claim 15 for use in the treatment of a disease selected from heartburn, indigestion, dyspepsia, erosive esophagitis, peptic ulcer, gastric ulcer, esophageal ulcers, esophagitis, laryngitis, pharyngitis, coarse voice, gastroesophageal reflux disease (GERD), Barrett's esophagus, gastric cancer, esophageal cancer, esophageal adenocarcinoma, gastritis and GERD-related pulmonary dysfunction, wherein the daily dose is 100 mg to 4000 mg and wherein the subject is in the fed state.

17. The gastro-retentive, oral dosage form of any one of claims 1-14 or the pharmaceutical composition of claim 15 for use in the treatment of a disease selected from heartburn, indigestion, dyspepsia, erosive esophagitis, peptic ulcer, gastric ulcer, esophageal ulcers, esophagitis, laryngitis, pharyngitis, coarse voice, gastroesophageal reflux disease (GERD), Barrett's esophagus, gastric cancer, esophageal cancer, esophageal adenocarcinoma, gastritis and GERD-related pulmonary dysfunction, wherein the total daily dose is 100 mg to 4000 mg.

18. The gastro-retentive, oral dosage form of any one of claims 1-14 or the pharmaceutical composition of claim 15 for use in the treatment of gastroesophageal reflux disease (GERD).

## Patentansprüche

1. Gastroretentive orale Dosierungsform zur Verwendung bei der Behandlung einer Erkrankung des oberen Gastrointestinaltrakts oder des Rachens, wobei die Darreichungsform in Form einer Tablette vorliegt und Folgendes umfasst:
a. ein Gallensäure-Sequestriermittel, ausgewählt aus Colesevelam und Colesevelam-Hydrochlorid, und
b. dispergiert in einer polymeren Matrix, die Poly(alkylen)oxid umfasst, zur verzögerten Freisetzung des Gallensäure-Sequestriermittels im Magen, wobei die polymere Matrix ein hydrophiles Poly(alkylen)oxid-Polymer umfasst, wobei die polymere Matrix ein hydrophiles Poly(alkylen)oxid-Polymer umfasst, so dass die Darreichungsform bei Aufnahme von Magenflüssigkeit auf eine Größe anschwillt, die ausreicht, um die Magenretention für eine Zeitspanne von 3 Stunden oder länger zu fördern, und wobei das Gallensäure-Sequestriermittel aus der Darreichungsform durch Erosion der polymeren Matrix über einen längeren Zeitraum von mindestens 3 Stunden freigesetzt wird, wobei die Freisetzungsrate des Arzneimittels aus der gastroretentiven Darreichungsform *in vitro* in Acetatpuffer bei pH 4,5 unter Verwendung einer USP Typ II- (Paddel) Apparatur gemessen wird, wobei die Tabletten in Senkern platziert werden.

2. Darreichungsform nach Anspruch 1, wobei die *in vitro* in Acetatpuffer bei einem pH-Wert von 4,5 unter Verwendung einer USP Typ II- (Paddle) Apparatur mit in Sinkern platzierten Tabletten gemessene Freisetzungsrate des Arzneimittels so ist, dass nach 4 Stunden nicht mehr als 40 % des Wirkstoffs aus der Darreichungsform freigesetzt worden sind.

3. Darreichungsform nach Anspruch 1 oder Anspruch 2, wobei die *in vitro* in Acetatpuffer bei einem pH-Wert von 4,5 unter Verwendung einer USP Typ II- (Paddle) Apparatur mit in Sinkern platzierten Tabletten gemessene Freisetzungsrate des Arzneimittels so ist, dass nach 16 Stunden zwischen 75-100 % des Wirkstoffs aus der Darreichungsform freigesetzt worden sind.

4. Darreichungsform nach einem der vorhergehenden Ansprüche, wobei die *in vitro* in Acetatpuffer bei einem pH-Wert von 4,5 unter Verwendung einer USP Typ II- (Paddle) Apparatur mit in Sinkern platzierten Tabletten gemessene Freisetzungsrate des Arzneimittels so ist, dass nach 16 Stunden zwischen 85-100 % des Wirkstoffs aus der Darreichungsform freigesetzt worden sind.

5. Darreichungsform nach einem der vorhergehenden Ansprüche, wobei die Dosis des Gallensäure-Sequestriermittels zwischen 400 mg und 600 mg liegt.

6. Darreichungsform nach Anspruch 6, wobei die Dosis des Gallensäure-Sequestriermittels 500 mg beträgt.

7. Darreichungsform nach einem der vorhergehenden Ansprüche, wobei das Poly(alkylen)oxid ein Poly(ethylen)oxid umfaßt.

8. Darreichungsform nach Anspruch 7, wobei das Poly(ethylen)oxid in einer Menge im Bereich von 40 Gewichtsprozent bis 75 Gewichtsprozent vorhanden ist.

9. Darreichungsform nach Anspruch 7, wobei das Poly(ethylen)oxid in einer Menge im Bereich von 40 Gewichtsprozent bis 60 Gewichtsprozent vorhanden ist.

10. Darreichungsform nach Anspruch 7, wobei das Poly(ethylen)oxid in einer Menge im Bereich von 45 Gewichtsprozent bis 55 Gewichtsprozent vorhanden ist.

11. Darreichungsform nach Anspruch 7, wobei das Poly(ethylen)oxid in einer Menge im Bereich von 40 Gewichtsprozent bis 50 Gewichtsprozent vorhanden ist.

12. Darreichungsform nach einem der vorhergehenden Ansprüche, die ferner eine Beschichtung umfaßt, wobei der Anteil der Beschichtung zwischen 5 % und 7,5 % (Gewicht:Gewicht) des Gesamttablettengewichts beträgt.

13. Darreichungsform nach einem der vorhergehenden Ansprüche, wobei die gastroretentive orale Darreichungsform innerhalb von 30 Minuten auf eine Größe anschwillt, die mindestens 110 % der ursprünglichen Größe beträgt.

14. Gastroretentive orale Dosierungsform nach einem der Ansprüche 1 bis 12, wobei die gastroretentive orale Darreichungsform innerhalb von 2 Stunden auf eine Größe anschwillt, die mindestens 140 % der ursprünglichen Größe beträgt.

15. Pharmazeutische Zusammensetzung, die die gastroretentive orale Dosierungsform nach einem der Ansprüche 1 bis 14 und ein zusätzliches therapeutisches Mittel umfasst.

16. Gastroretentive orale Darreichungsform nach einem der Ansprüche 1 bis 14 oder pharmazeutische Zusammensetzung nach Anspruch 15 zur Verwendung bei der Behandlung einer Krankheit, ausgewählt aus Sodbrennen, Verdauungsstörungen, Dyspepsie, erosiver Ösophagitis, Ulcus pepticum, Magengeschwür, Ösophagusgeschwüren, Ösophagitis, Laryngitis, Pharyngitis, rauer Stimme, gastroösophagealer Refluxkrankheit (GastroEsophageal Reflux Disease, GERD), Barrett-Ösophagus, Magenkrebs, Speiseröhrenkrebs, Adenokarzinom der Speiseröhre, Gastritis und GERO-bedingter Lungenfunktionsstörung, wobei die Tagesdosis 100 mg bis 4000 mg beträgt und wobei sich der Proband im ernährten Zustand befindet.

17. Gastroretentive orale Darreichungsform nach einem der Ansprüche 1 bis 14 oder pharmazeutische Zusammensetzung nach Anspruch 15 zur Verwendung bei der Behandlung einer Krankheit, ausgewählt aus Sodbrennen, Verdauungsstörungen, Dyspepsie, erosiver Ösophagitis, Ulcus pepticum, Magengeschwür, Ösophagusgeschwüren, Ösophagitis, Laryngitis, Pharyngitis, rauer Stimme, gastroösophagealer Refluxkrankheit (GastroEsophageal Reflux Disease, GERD), Barrett-Ösophagus, Magenkrebs, Speiseröhrenkrebs, Adenokarzinom der Speiseröhre, Gastritis und GERO-bedingter Lungenfunktionsstörung, wobei die Gesamttagesdosis 100 mg bis 4000 mg beträgt.

18. Gastroretentive orale Darreichungsform nach einem der Ansprüche 1 bis 14 oder pharmazeutische Zusammensetzung nach Anspruch 15 zur Verwendung bei der Behandlung der gastroösophagealen Refluxkrankheit (GERD).

## Revendications

1. Forme galénique orale à rétention gastrique à utiliser dans le traitement d'une maladie du tractus gastro-intestinal supérieur ou de la gorge, dans laquelle ladite forme galénique est sous la forme d'un comprimé comprenant :
a. un séquestrant des acides biliaires choisi parmi le colesevelam et le chlorhydrate de colesevelam, et
b. dispersé dans une matrice polymère comprenant du poly(alkylène)oxyde, pour une libération prolongée du séquestrant des acides biliaires dans l'estomac, dans laquelle la matrice polymère comprend un polymère hydrophile de poly(alkylène)oxyde de telle sorte que, lors des imbibitions de fluide gastrique, ladite forme galénique gonfle à une taille suffisante pour favoriser la rétention gastrique pendant une période de temps de 3 heures ou plus et dans laquelle le séquestrant des acides biliaires est libéré de la forme galénique par érosion de la matrice polymère sur une période de temps prolongée d'au moins 3 heures, dans laquelle la vitesse de libération du médicament à partir de la forme galénique à rétention gastrique est mesurée in vitro dans un tampon acétate à pH 4,5, en utilisant un appareil USP de type II (palette) avec les comprimés placés dans des platines.

2. Forme galénique selon la revendication 1, dans laquelle la vitesse de libération du médicament mesurée in vitro, dans un tampon acétate à pH 4,5, en utilisant un appareil USP de type II (palette) avec les comprimés placés dans des platines est telle que pas plus de 40% du médicament soit libéré de la forme galénique après 4 heures.

3. Forme galénique selon la revendication 1 ou 2, dans laquelle la vitesse de libération du médicament mesurée *in vitro,* dans un tampon acétate à pH 4,5, en utilisant un appareil USP Type II (palette) avec les comprimés placés dans des platines est telle qu'entre 75 à 100% du médicament soit libéré de la forme galénique après 16 heures.

4. Forme galénique selon l'une quelconque des revendications précédentes, dans laquelle la vitesse de libération du médicament mesurée in vitro, dans un tampon acétate à pH 4,5, en utilisant un appareil USP de type II (palette) avec les comprimés placés dans des platines est telle qu'entre 85 à 100% du médicament soit libéré de la forme galénique après 16 heures.

5. Forme galénique selon l'une quelconque des revendications précédentes, dans laquelle la dose de séquestrant des acides biliaires est comprise entre 400 mg et 600 mg.

6. Forme galénique selon la revendication 6, dans laquelle la dose de séquestrant des acides biliaires est de 500 mg.

7. Forme galénique selon l'une quelconque des revendications précédentes, dans laquelle ledit poly(alkylène)oxyde comprend un poly(éthylène)oxyde.

8. Forme galénique selon la revendication 7, dans laquelle le poly(éthylène)oxyde est présent en une quantité allant d'un rapport de 40 pour cent en poids à 75 pour cent en poids.

9. Forme galénique selon la revendication 7, dans laquelle le poly(éthylène)oxyde est présent en une quantité allant d'un rapport de 40 pour cent en poids à un rapport de 60 pour cent en poids.

10. Forme galénique selon la revendication 7, dans laquelle le poly(éthylène)oxyde est présent en une quantité allant de 45 pour cent en poids à 55 pour cent en poids.

11. Forme galénique selon la revendication 7, dans laquelle le poly(éthylène)oxyde est présent en une quantité allant de 40 pour cent en poids à 50 pour cent en poids.

12. Forme galénique selon l'une quelconque des revendications précédentes, comprenant en outre un enrobage, dans laquelle le niveau d'enrobage est compris entre 5% et 7,5% (poids : poids) du poids total du comprimé.

13. Forme galénique selon l'une quelconque des revendications précédentes, dans laquelle ladite forme galénique orale à rétention gastrique gonfle jusqu'à une taille qui est au moins 110% de la taille d'origine en 30 minutes.

14. Forme galénique orale à rétention gastrique selon l'une quelconque des revendications 1 à 12, dans laquelle ladite forme galénique orale à rétention gastrique gonfle jusqu'à une taille qui est au moins 140% de la taille d'origine en 2 heures.

15. Composition pharmaceutique comprenant la forme galénique orale à rétention gastrique selon l'une quelconque des revendications 1 à 14 et un agent thérapeutique supplémentaire.

16. Forme galénique orale à rétention gastrique selon l'une quelconque des revendications 1 à 14 ou composition pharmaceutique selon la revendication 15 à utiliser dans le traitement d'une maladie choisie parmi les brûlures d'estomac, l'indigestion, la dyspepsie, l'œsophagite érosive, l'ulcère gastroduodénal, l'ulcère gastrique, les ulcères œsophagiens, l'œsophagite, la laryngite, la pharyngite, la voix rauque, le reflux gastro-œsophagien (RGO), l'œsophage de Barrett, le cancer gastrique, le cancer œsophagien, l'adénocarcinome œsophagien, la gastrite et la dysfonction pulmonaire liée au RGO, dans laquelle la dose quotidienne est de 100 mg à 4000 mg et dans laquelle le sujet est à l'état nourri.

17. Forme galénique orale à rétention gastrique selon l'une quelconque des revendications 1 à 14 ou composition pharmaceutique selon la revendication 15 à utiliser dans le traitement d'une maladie choisie parmi les brûlures d'estomac, l'indigestion, la dyspepsie, l'œsophagite érosive, l'ulcère gastroduodénal, l'ulcère gastrique, les ulcères œsophagiens, l'œsophagite, la laryngite, la pharyngite, la voix rauque, le reflux gastro-œsophagien (RGO), l'œsophage de Barrett, le cancer gastrique, le cancer œsophagien, l'adénocarcinome œsophagien, la gastrite et la dysfonction pulmonaire liée au RGO, dans laquelle la dose quotidienne est de 100 mg à 4000 mg.

18. Forme galénique orale à rétention gastrique selon l'une quelconque des revendications 1 à 14 ou composition pharmaceutique selon la revendication 15 à utiliser dans le traitement du reflux gastro-œsophagien (RGO).
